# EUROPEAN PATENT APPLICATION

(11) **EP 4 328 313 A1**
(43) Date of publication of application: **28.02.2024**
(21) Application number: 21937868.4
(22) Date of filing: 21.04.2021
(51) Int. Cl.: C12N 15/864, A61K 35/76, A61K 38/45, A61K 48/00, A61P 43/00

(54) **ADENO-ASSOCIATED VIRUS VIRION FOR TREATING ORNITHINE TRANSCARBAMYLASE DEFICIENCY**

(71) Applicant: Jichi Medical University, Tochigi 329-0498 (JP); Gene Therapy Research Institution Co., Ltd., Kawasaki-ku Kawasaki-shi Kanagawa 2100821 (JP)
(72) Inventor: MURAMATSU, Shin-ichi, Shimotsuke-shi, Tochigi 329-0498 (JP); TAKINO, Naomi, Shimotsuke-shi, Tochigi 329-0498 (JP); ITO, Mika, Shimotsuke-shi, Tochigi 329-0498 (JP); YAMAGATA, Takanori, Shimotsuke-shi, Tochigi 329-0498 (JP); MURAMATSU, Kazuhiro, Shimotsuke-shi, Tochigi 329-0498 (JP)
(74) Representative: Elkington and Fife LLP
(86) International application number: PCT/JP2021/016141
(87) International publication number: WO 2022/224372

(57) **Abstract**

The present application provides a novel means for treating ornithine transcarbamylase deficiency. More specifically, the present application provides a modified adeno-associated virus vector which expresses ornithine transcarbamylase so that an attack from a neutralizing antibody in blood is reduced and a gene is efficiently introduced into a liver of a living body (for example, human). The present application provides, for example, a modified adeno-associated virus vector which expresses ornithine transcarbamylase, which contains a modified VP1 protein that contains, for example, an amino acid sequence obtained by substituting at least one of the amino acids at positions 472, 587 and 706 in the amino acid sequence of the VP1 protein with another amino acid, and which does not cross-react with a neutralizing antibody against AAV2.

## Description

### TECHNICAL FIELD

The present invention relates to a recombinant adeno-associated virus (rAAV) vector for the treatment of urea cycle disorder, which is less susceptible to neutralizing antibodies in a serum. More particularly, the present invention relates to a modified rAAV capable of expressing recombinant ornithine transcarbamylase (OTC), having reduced cross-reactivity with neutralizing antibodies in the serum of a living body, and highly efficient in transferring a gene into the liver of a living body (e.g., human), and to a pharmaceutical composition containing said rAAV.

### BACKGROUND ART

Deficiency of ornithine transcarbamylase (OTC), an enzyme of the urea cycle involved in the metabolism of ammonia, is caused by a loss-of-function mutation in the OTC gene on the X chromosome. OTC deficiency is the most frequent urea cycle disorder. While the OTC deficiency is an X-linked genetic disorder, women also present a variety of symptoms. Hyperammonemia is caused by increased catabolism (fever, fasting, etc.), excessive protein intake, and the like. Clinical manifestations include vomiting, poor suckling, tachypnea, cramps, impaired consciousness, behavioral abnormalities, and developmental disabilities. In boys, neonatal onset is common and can be fatal. In girls, the disorder may be found due to liver dysfunction. Lifelong treatment with a low-protein diet, and administrations of a residual nitrogen excretion enhancer and arginine hydrochloride are necessary. In addition, hemodialysis and filtration therapy is necessary in the acute stage, and a living donor liver transplantation is employed in cases of repeated episodes of hyperammonemia. Even after the treatment is initiated, effects on the central nervous system will be unavoidable, and the accompanying intellectual disability often forces the patient to lead a life that is not socially independent. From the above background, development of an effective treatment is an urgent issue (Non-patent literature 1).

Gene transfer vectors adopting adeno-associated viruses (AAVs) can transfer genes into cells in the body such as nerve cells, hepatocytes (hepatic parenchymal cells), retinal cells, muscle cells, myocardial cells, vascular endothelial cells, and adipocytes, and allow the expression for a long time (Patent literature 1-3). Therefore, they have been clinically applied as vectors for the gene therapies for hemophilia, retinitis pigmentosa, Parkinson's disease, and the like (Non-patent literature 2 and 3). Recently, such vectors are also frequently used as vectors for the transfer of sgRNA and gene of CAS9 protein for gene editing (Patent literature 3, and Non-patent literature 4). For hemophilia, a gene therapy in which an AAV vector that can express factor VIII or factor IX is transferred into hepatocytes is reported to give promising results (Patent literature 4, and Non-patent literature 5 and 6).

In the gene therapy for OTC deficiency described above, adeno-associated virus serotype 8 (AAV8) vector has been applied to human liver as a target organ (Non-patent literature 7). However, while the AAV8 vector is highly efficient in transferring a gene into a mouse liver, its efficiency is low in transferring a gene into a human liver. Moreover, AAV3B, which is said to be more efficient than AAV8 in transferring a gene into human hepatocytes, cross-reacts with a neutralizing antibody against AAV2, which is present in more than 80% of adults (Non-patent literature 8-10), and thus it is not expected to be effective in many patients. In addition, a gene therapy for patients who had not been eligible for a gene therapy because they had neutralizing antibodies, a repeated gene therapy for patients who did not have an adequate response to the first round of gene therapy, etc. are underway.

### PRIOR ART LITERATURE

### Patent literature

Patent literature 1: International Patent Application Publication WO2008/124724
Patent literature 2: International Patent Application Publication WO2012/057363
Patent literature 3: International Patent Application Publication WO2018/131551
Patent literature 4: Japanese Unexamined Patent Application Publication (translation of PCT) No. 2016-525356

### Non-patent literature

Non-patent literature 1: Nakamura K, et al., Pediatr Int.; 56(4): 506-9, 2014
Non-patent literature 2: Dunber CE, et al., Science 359: eaan4672, 2018
Non-patent literature 3: Hastie E, Samulski RJ, Hum Gene Ther 26: 257-265, 2015
Non-patent literature 4: Ohmori T, et al., Sci Rep 7: 4159, 2017
Non-patent literature 5: George LA, et al., N Engl J Med 377: 2215-2227, 2017
Non-patent literature 6: Rangarajan S, et al., N Engl J Med 377: 2519-2530, 2017
Non-patent literature 7: Wang L., et al., Gene Therapy (2012) 19, 404-410
Non-patent literature 8: Mimuro J, et al., J Med Virol 86: 1990-1997, 2014
Non-patent literature 9: Ling C, et al., J Integr Med 13: 341-346, 2015
Non-patent literature 10: Meliani A, et al., Hum Gene Ther Methods 26: 45-53, 2015

### SUMMARY OF INVENTION

### Problems to be solved by the invention

Therefore, there is a need to establish a therapeutic means for diseases caused by genomic disorders of hepatocytes (e.g., ornithine transcarbamylase deficiency) by using an AAV vector which has reduced cross-reactivity with neutralizing antibodies against AAV2, etc. in a serum and which is highly efficient in transferring a gene into hepatocytes, for example, a novel AAV vector derived from AAV3B or AAV8.

### Means for solving the problems

In order to solve the above problem, the present inventors have created a modified AAV vector that expresses ornithine transcarbamylase and has reduced cross-reactivity with neutralizing antibodies (such as AAV2 neutralizing antibody) in a serum, thereby accomplishing the present invention.

Specifically, the present invention provides the inventions exemplified below, which include an AAV vector for expressing ornithine transcarbamylase which has reduced cross-reactivity with neutralizing antibodies in a serum and which is highly efficient in transferring a gene into hepatocytes, such as an rAAV vector for a gene therapy targeting hepatocytes, a pharmaceutical composition comprising the same, or the like.
[1] An adeno-associated virus vector comprising:
   a capsid protein containing an amino acid sequence in which at least one of serine at position 472, serine at position 587, and asparagine at position 706 in the amino acid sequence represented by SEQ ID NO:2 or 3 is substituted with another amino acid, or an amino acid sequence in which 1-6 residues in the substituted amino acid sequence are deleted, substituted, inserted, and/or added, in addition to the residues at positions 472, 587, and 706; and
   a polynucleotide encoding a protein that contains the amino acid sequence represented by SEQ ID NO: 11 or an amino acid sequence having 90% or more identity to the amino acid sequence represented by SEQ ID NO: 11, and that has ornithine transcarbamylase activity,
   wherein the adeno-associated virus vector does not cross-react at least with a neutralizing antibody against AAV serotype 2.
[2] The adeno-associated virus vector according to [1] above, comprising a capsid protein having an amino acid sequence in which each of the serine at position 472, the serine at position 587, and the asparagine at position 706 is substituted with an amino acid selected from the group consisting of glycine, alanine, valine, leucine, threonine, and isoleucine.
[3] The adeno-associated virus vector according to [1] above, comprising a capsid protein having an amino acid sequence in which at least one of the serine at position 472, the serine at position 587, and the asparagine at position 706 is substituted with alanine.
[4] The adeno-associated virus vector according to [1] above, wherein the capsid protein comprises a protein having the amino acid sequence represented by SEQ ID NO:4.
[5] The adeno-associated virus vector according to [1] above, wherein the neutralizing antibody is an antibody against an adeno-associated virus of a serotype different from AAV3 (e.g., AAV3B) or AAV8.
[6] The adeno-associated virus vector according to [1] above, wherein the neutralizing antibody is an antibody against AAV2.
[7] The adeno-associated virus vector according to [1] above, comprising a virus genome containing a hepatocyte-specific promoter sequence.
[8] The adeno-associated virus vector according to [1] above, wherein the hepatocyte-specific promoter sequence comprises a promoter selected from the group consisting of an ApoE promoter, an antitrypsin promoter, a cKit promoter, a promoter for a liver-specific transcription factor (HNF-1, HNF-2, HNF-3, HNF-6, C/ERP, DBP), an albumin promoter, a promoter for a thyroxine-binding globulin (TBG), and a HCRhAAT promoter, or a promoter which contains a polynucleotide sequence having 90% or more homology with these promoters and which functions in a liver-specific manner.
[9] An adeno-associated virus vector comprising:
   a capsid protein containing an amino acid sequence in which at least one of serine at position 472, serine at position 587, and asparagine at position 706 in the amino acid sequence represented by SEQ ID NO:2 or 3 is substituted with another amino acid, or an amino acid sequence in which 1-6 residues in the substituted amino acid sequence are deleted, substituted, inserted, and/or added, in addition to the residues at positions 472, 587, and 706; and
   a polynucleotide encoding a protein that contains the amino acid sequence represented by SEQ ID NO: 11 or an amino acid sequence having 90% or more identity to the amino acid sequence represented by SEQ ID NO: 11, and that has ornithine transcarbamylase activity.
[10] A polynucleotide, encoding any one of the following sequences:
   an amino acid sequence in which at least one of serine at position 472, serine at position 587, and asparagine at position 706 in the amino acid sequence represented by SEQ ID NO:2 or 3 is substituted with another amino acid, and 1-6 amino acid residues at other residue positions are deleted, substituted, inserted, or added;
   an amino acid sequence in which each of the serine at position 472, the serine at position 587, and the asparagine at position 706 is substituted with an amino acid selected from the group consisting of glycine, alanine, valine, leucine, threonine, and isoleucine;
   an amino acid sequence in which at least one of the serine at position 472, the serine at position 587, and the asparagine at position 706 is substituted with alanine; and
   the amino acid sequence represented by SEQ ID NO:4.
[11] A pharmaceutical composition for transferring a gene into the liver of a living body, comprising the adeno-associated virus vector according to any one of [1] to [9] above.
[12] The pharmaceutical composition according to [11] above, wherein the living body is human.
[13] The pharmaceutical composition according to [11] or [12] above, which is used for the treatment of ornithine transcarbamylase deficiency.
[14] The pharmaceutical composition according to [11] or [12] above, which is used for reducing ammonia level in the blood.

### EFFECT OF THE INVENTION

The present invention provides an AAV vector that expresses recombinant ornithine transcarbamylase (OTC), has reduced cross-reactivity with a neutralizing antibody against AAV2 or the other, and is highly efficient in transferring a gene into hepatocytes, such as AAV vectors derived from AAV3B, AAV8, etc. Furthermore, the AAV vector according to the present invention can be used to perform a gene therapy to patients who had not been eligible for a gene therapy because they inherently had neutralizing antibodies, and to repeat a gene therapy to patients who did not have an adequate response to the first round of gene therapy. The AAV vector of the present invention is especially useful for the treatment of ornithine transcarbamylase deficiency particularly because it can improve the transfer of ornithine transcarbamylase gene into hepatocytes.

### BRIEF DESCRIPTION OF DRAWINGS

[Figure 1A] Figure 1A shows the amino acid alignment of VP1 proteins of AAV3A, AAV3B, and AAV.GT5.
[Figure 1B] Figure 1B shows the alignment continued from Figure 1A.
[Figure 1C] Figure 1C shows the alignment of the amino acid sequences of Rep proteins of AAV3A, AAV3B, and AAV.GT5 (referred to as ARep, BRep, and baRep, respectively).
[Figure 1D] Figure 1D shows the alignment continued from Figure 1C.
[Figure 2A] Figure 2A shows a GFP expression level obtained with AAV.GT5 in human liver-derived HepG2 cells.
[Figure 2B] Figure 2B shows the states of the gene-transferred, GFP-expressing cells shown in Figure 2A.
[Figure 2C] Figure 2C shows a luciferase expression level obtained with AAV.GT5 in human liver-derived HepG2 cells.
[Figure 3A] Figure 3A shows a GFP expression level obtained with AAV.GT5 in human liver-derived PXB cells.
[Figure 3B] Figure 3B shows the states of the gene-transferred, GFP-expressing cells shown in Figure 3A.
[Figure 4A] Figure 4A shows comparative results of the luciferase expression levels in HEK293 cells obtained with the AAV vectors after the reaction with a serum containing a neutralizing antibody against AAV (Serum #01).
[Figure 4B] Figure 4B shows comparative results of the luciferase expression levels in HEK293 cells obtained with the AAV vectors after the reaction with a serum containing a neutralizing antibody against AAV (Serum #02).
[Figure 4C] Figure 4C shows comparative results of the luciferase expression levels in HEK293 cells obtained with the AAV vectors after the reaction with a serum containing a neutralizing antibody against AAV (Serum #03).
[Figure 4D] Figure 4D shows comparative results of the luciferase expression levels in HEK293 cells obtained with the AAV vectors after the reaction with a serum containing a neutralizing antibody against AAV (Serum #04).
[Figure 4E] Figure 4E shows comparative results of the luciferase expression levels in HEK293 cells obtained with the AAV vectors after the reaction with a serum containing a neutralizing antibody against AAV (Serum #05).
[Figure 4F] Figure 4F shows comparative results of the luciferase expression levels in HEK293 cells obtained with the AAV vectors after the reaction with a serum containing a neutralizing antibody against AAV (Serum #06).
[Figure 4G] Figure 4G shows comparative results of the luciferase expression levels in HEK293 cells obtained with the AAV vectors after the reaction with a serum containing a neutralizing antibody against AAV (Serum #07).
[Figure 4H] Figure 4H shows comparative results of the luciferase expression levels in HEK293 cells obtained with the AAV vectors after the reaction with a serum containing a neutralizing antibody against AAV (Serum #08).
[Figure 4I] Figure 4I shows comparative results of the luciferase expression levels in HEK293 cells obtained with the AAV vectors after the reaction with a serum containing a neutralizing antibody against AAV (Serum #09).
[Figure 4J] Figure 4J shows comparative results of the luciferase expression levels in HEK293 cells obtained with the AAV vectors after the reaction with a serum containing a neutralizing antibody against AAV (Serum #10).
[Figure 4K] Figure 4K shows comparative results of the GFP expression levels obtained with the AAV vectors after the reaction with sera containing neutralizing antibodies against AAV (Serum #A-#D).
[Figure 5A] Figure 5A shows the configuration of the recombinant virus genome AAV.GT5-HCRhAAT promoter-human OTC-WPRE.
[Figure 5B] (Continued from Figure 5A)
[Figure 6] Figure 6 shows expression levels of recombinant OTC mRNA in mouse PBX cells.
[Figure 7] Figure 7 shows expression levels of recombinant OTC proteins in mouse PBX cells.
[Figure 8] Figure 8 shows activities of recombinant OTC proteins in mouse PBX cells.

### MODES FOR CARRYING OUT INVENTION

The present invention provides a recombinant adeno-associated virus vector that expresses recombinant ornithine transcarbamylase (OTC) and that improves the efficiency of gene transfer into liver cells, a pharmaceutical composition containing said vector, and the like.

### 1. rAAV vector of the present invention

### 1.1. Adeno-associated virus

Adeno-associated viruses (AAVs) include viruses of many known serotypes. Examples of AAVs that present tropism towards hepatocytes (hepatic parenchymal cells) include the viruses of serotypes 2, 3 (3A and 3B), and 8. According to the present invention, a vector used for delivery to hepatocytes of a living body may be, for example, any of the various AAV vectors described in Patent literature 1 (WO 2008/124724).

Native AAVs are nonpathogenic. Taking advantage of this feature, various recombinant virus vectors carrying a gene of interest have been prepared and used for gene therapies (see, for example, WO2003/018821, WO2003/053476, WO2007/001010, YAKUGAKU ZASSHI, 126 (11), 1021-1028, etc.). Wild-type AAV genome is a single-stranded DNA molecule with a total nucleotide length of about 5 kb, and is either a sense or antisense strand. An AAV genome generally has inverted terminal repeat (ITR) sequences of approximately 145 nucleotides in length at both 5' and 3' ends of the genome. The ITRs are known to have various functions, including a function as the origin of replication of the AAV genome, a function as the signal for packaging the genome into virions, and the like (see, for example, YAKUGAKU ZASSHI 126 (11) 1021-1028, mentioned above, etc.). The internal domain of the wild-type AAV genome (hereinafter, internal domain) flanked by ITRs contains AAV replication (rep) gene and capsid (cap) gene. These rep and cap genes encode a protein involved in virus replication (Rep) and a capsid protein forming a virus particle, i.e., an icosahedral outer shell, (e.g., at least one of VP1, VP2, and VP3), respectively. For further detail, see, for example, Human Gene Therapy, 13, pp. 345-354, 2002, Neuronal Development 45, pp. 92-103, 2001, Experimental Medicine 20, pp. 1296-1300, 2002, YAKUGAKU ZASSHI 126 (11), 1021-1028, Hum Gene Ther, 16, 541-550, 2005, etc.

The rAAV vectors of the present invention include, but are not limited to, vectors derived from native adeno-associated virus serotype 1 (AAV1), serotype 2 (AAV2), serotype 3 (AAV3A/AAV3B), serotype 4 (AAV4), serotype 5 (AAV5), serotype 6 (AAV6), serotype 7 (AAV7), serotype 8 (AAV8), serotype 9 (AAV9), serotype 10 (AAV10), and serotype RH10 (AAVrh10; Hu, C. et al., Molecular Therapy vol. 22, no. 10, Oct. 2014, 1792-1802). Nucleotide sequences of these AAV genomes are known and reference can be made to the nucleotide sequences with the registered GenBank accession numbers of: AF063497.1 (AAV1), AF043303 (AAV2), NC_001729 (AAV3), NC_001829.1 (AAV4), NC_006152.1 (AAV5), AF028704.1 (AAV6), NC_006260.1 (AAV7), NC_006261.1 (AAV8), AY530579 (AAV9), and AY631965.1 (AAV10), respectively.

According to the present invention, a capsid protein (VP1, VP2, VP3, etc.) derived from AAV2, AAV3B (AF028705.1), AAV8, or AAV9 is preferably utilized, especially for their tropism towards hepatocytes. The amino acid sequences of these capsid proteins are known and reference can be made, for example, to the sequences registered under the above-mentioned GenBank accession numbers corresponding to the respective AAVs.

### 1.2. Capsid protein of the present invention

An rAAV vector used in the present invention comprises a mutated (modified) capsid protein. Such a mutant capsid protein comprises a capsid protein having a mutated amino acid sequence in which at least one (e.g., one, preferably two, and more preferably all three) of serine at position 472, serine at position 587, and asparagine at position 706 in the amino acid sequence represented by SEQ ID NO:2 or 3 is substituted with other amino acid, and further a mutant protein which can serve as a capsid protein and which contains an amino acid sequence having deletion, substitution, insertion, and/or addition of a plurality of amino acid residues at positions different from the residue positions 472, 587 and 706 in said mutated amino acid sequence. Two or more of these deletion, substitution, insertion, and/or addition may be contained together in combination.

Moreover, the rAAV vector used in the present invention comprises a capsid protein having an amino acid sequence in which at least one (e.g., one, preferably two, and more preferably all three) of serine at position 472, serine at position 587, and asparagine at position 706 in the amino acid sequence represented by SEQ ID NO:2 or 3 is substituted with an amino acid selected from the group consisting of glycine, alanine, valine, leucine, threonine, and isoleucine, preferably with alanine (e.g., the amino acid sequence represented by SEQ ID NO:4), and further a capsid protein containing an amino acid sequence which includes deletion, substitution, insertion, and/or addition of a plurality of amino acid residues at positions different from the residue positions 472, 587 and 706 in said mutated amino acid sequence. Two or more types of these deletion, substitution, insertion, and addition may be contained together in combination.

The number of deletion, substitution, insertion, and/or addition of the above amino acid residues is, for example, 1-74, 1-70, 1-60, 1-50, 1-40, 1-35, 1-30, 1-25, 1-20, 1-15, 1-14, 1-13, 1-12, 1-11, 1-10, 1-9 (1 to several), 1-8, 1-7, 1-6, 1-5, 1-4, 1-3, 1-2, or 1. In general, the smaller the number of deletion, substitution, insertion, and/or addition of the above amino acid residues, the better.

Preferably, the mutant capsid protein of the rAAV vector used in the present invention may be a protein which can serve as a capsid protein and which has an amino acid sequence with about 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more identity to any of the amino acid sequences represented by SEQ ID NOs:2-4 (735-738 residues).

According to the present invention, a protein that can serve as a capsid protein refers to a protein which can form a virus vector capable of infecting a target cell. The capsid protein used in the present invention can form a virus vector by itself or together with other capsid protein member (e.g., VP2, VP3, etc.). A polynucleotide including a therapeutic gene of interest that is to be delivered to target cells, for example, hepatocytes, is packaged into this virus vector.

Preferably, a virus vector containing a mutant capsid protein has an infectivity comparable to or more than that of a virus vector containing a wild-type capsid protein, meaning specifically that the infectivity is preferably 50%, 60%, 70%, 80%, 85%, 90%, 91%, 92%, 93%, 94%, 95%, 96%, 97%, 98%, 99% or more relative to the infectivity of a wild-type virus genome by weight. The infectivity can be measured by a method known in the art, for example, by a reporter assay using a reporter such as β-galactosidase, GFP protein, or luciferase.

Examples of the amino acid residues that are mutually replaceable in the protein (polypeptide) of the present invention are shown below. Amino acid residues belonging to the same group are replaceable with each other.
Group A: Leucine, isoleucine, norleucine, valine, norvaline, alanine, 2-aminobutanoic acid, methionine, o-methyl serine, t-butyl glycine, t-butyl alanine, cyclohexylalanine;
Group B: Aspartic acid, glutamic acid, isoaspartic acid, isoglutamic acid, 2-aminoadipic acid, 2-aminosuberic acid;
Group C: Asparagine, glutamine;
Group D: Lysine, arginine, ornithine, 2,4-diaminobutanoic acid, 2,3-diaminopropionic acid;
Group E: Proline, 3-hydroxyproline, 4-hydroxyproline;
Group F: Serine, threonine, homoserine;
Group G: Phenylalanine, tyrosine.

A protein containing an amino acid residue substitution of interest can be prepared according to a technique known to those skilled in the art, for example, by a common genetic engineering technique or chemical synthesis. For such a genetic engineering procedure, see, for example, Molecular Cloning 4th Edition, J. Sambrook et al., Cold Spring Harbor Lab. Press. 2012, Current Protocols in Molecular Biology, John Wiley and Sons 1987-2018 (ISSN: 1934-3647, etc.), and the like.

The rAAV vector used in the present invention may contain, in the packaged genome or in a genome of a helper virus, a gene coding for the Rep protein involved in replication. Such a Rep protein may have an amino acid sequence that preferably have about 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more identity to the wild-type Rep protein provided that it has the function of recognizing the ITR sequences to carry out genome replication depending on said sequences so that the rAAV of the present invention is replicated, the function of packaging the wild-type AAV genome (or the rAAV genome) into the virus vector, and the function of forming a rAAV vector of the present invention which are comparable to the wild-type Rep protein. Alternatively, it may include deletion, substitution, insertion, and/or addition of 60 or less, 50 or less, 40 or less, 30 or less, 20 or less, 15 or less, 10 or less or 5 or less amino acid residues. Herein, "comparable to the wild-type" means that the specific activity relative to the wild type is 50%, 60%, 70%, 80%, 90% or more. According to the present invention, a Rep protein from a known AAV3, for example, a Rep protein from AAV3A or AAV3B, or a fusion protein thereof (the amino acid sequence represented by SEQ ID NO:8) or the like can be preferably used, but not limited thereto.

In one embodiment of the present invention, the above-described capsid protein VP1 or the like (VP1, VP2, and/or VP3) and Rep protein encoded in the internal domain of the wild-type AAV genome are used by incorporating polynucleotides coding for these proteins into an AAV helper plasmid, thereby obtaining a rAAV of the present invention. If necessary, the capsid protein (VP1, VP2, and/or VP3) and the Rep protein used in the present invention may be incorporated into one, two, three or more types of plasmids. Optionally, one or more types of these capsid protein and Rep protein may be contained in the AAV genome. According to the present invention, the capsid protein (VP1, VP2, and/or VP3) and the Rep protein are preferably all encoded together by one polynucleotide to be provided as an AAV helper plasmid.

### 1.3. Virus Genome

### (1) rAAV genome

The polynucleotide packaged into the AAV vector of the present invention (i.e., the polynucleotide) can be prepared by substituting the polynucleotide of an internal domain (i.e., either or both rep gene and cap gene) located between the ITRs at the 5' and 3' ends of the wild-type genome with a gene cassette containing a polynucleotide encoding the protein of interest (genome editing means and/or repair gene), a promoter sequence, etc. for transcribing said polynucleotide. Preferably, the 5' and 3' ITRs are located at the 5' and 3' terminals of the AAV genome, respectively. The ITRs at the 5' and 3' terminals of the rAAV genome of the present invention preferably include, but is not specifically limited to, the 5' ITR and the 3' ITR contained in the genome of AAV1, AAV2, AAV3, AAV6, AAV8, or AAV9. Since the ITR parts generally have easily altered complementary sequences (flip and flop structure), the orientation of the 5' and 3' ITRs contained in the rAAV genome of the present invention may be inverted. In the rAAV genome of the present invention, the polynucleotide that replaces the internal domain (namely, the genome editing means and/or the repair gene) preferably has a practical length that is substantially equal to the length of the original polynucleotide. Specifically, the total length of the rAAV genome of the present invention is substantially equal to the total length of the wild-type, i.e., 5 kb, for example, about 2-6 kb, preferably about 4-6 kb. The length of the therapeutic gene incorporated into the rAAV genome of the present invention, excluding the length of the transcriptional regulatory region including the promoter, the polyadenylation site and others (assuming, for example, about 1-1.5 kb), is preferably, but not limited to, about 0.01-3.7 kb, more preferably about 0.01-2.5 kb, and still more preferably 0.01-2 kb. Furthermore, as long as the total length of the rAAV genome is within the above-mentioned range, two or more kinds of therapeutic genes can be incorporated together using a known technique such as interposition of a known internal ribosome entry site (IRES) sequence, T2A sequence, etc. When the rAAV of the present invention expresses two or more kinds of proteins, the genes encoding these proteins may be oriented in the same or different directions.

In general, if the polynucleotide packaged into the rAAV vector is a single strand, it may take times (several days) until the gene of interest (therapeutic gene, etc.) is expressed. In this case, the gene of interest to be introduced is designed to have a self-complementary (sc) type structure to shorten the time for expression. Specifically, see, for example, Foust K.D. et al. (Nat Biotechnol., 2009 Jan; 27(1): 59-65), etc. The polynucleotide packaged into the AAV vector of the present invention may have either a non-sc type structure or a sc type structure. Preferably, the polynucleotide packaged into the AAV vector of the present invention has a non-sc type structure.

The capsid protein used in the present invention may be encoded, for example, by a polynucleotide which is suitably modified based on the codon preference in the host cell. The polynucleotide encoding a preferred capsid protein used in the present invention comprises, for example, a polynucleotide which has deletion, substitution, insertion, and/or addition of one or more (e.g., 1-50, 1-40, 1-30, 1-25, 1-20, 1-15, 1-10, 1-9 (one to several), 1-8, 1-7, 1-6, 1-5, 1-4, 1-3, 1-2, 1, etc.) nucleotides in a polynucleotide sequence coding for any of the amino acid sequences represented by SEQ ID NOs:2-4, a polynucleotide coding for a protein containing any of the amino acid sequences represented by SEQ ID NOs:2-4, or a protein including deletion, substitution, insertion, and/or addition of above-mentioned one or more amino acids in any of the amino acid sequences represented by SEQ ID NOs:2-4 and capable of serving as a capsid protein (i.e., which can form a capsomere). Two or more of these deletion, substitution, insertion, and/or addition can be included together in combination. In general, the smaller the number of the above-described deleted, substituted, inserted, and/or added nucleotides, the better. Moreover, a preferred polynucleotide according to the present invention is, for example, a polynucleotide which can hybridize to a polynucleotide coding for any of the amino acid sequences represented by SEQ ID NOs:2-4 or to a complementary sequence thereof under stringent hybridization conditions, the polynucleotide coding for a protein containing any of the amino acid sequences represented by SEQ ID NOs:2-4 or a capsid protein including deletion, substitution, insertion, and/or addition of above-mentioned one or more amino acids in the amino acid sequences represented by SEQ ID NOs:2-4.

Hybridization can be carried out according to a known method or a method corresponding thereto, for example, according to a method described in Molecular Cloning (Molecular Cloning 4th Edition, J. Sambrook et al., Cold Spring Harbor Lab. Press. 2012). When a commercially available library is employed, hybridization can be carried out, for example, according to the method described in the instruction provided by the manufacturer. Herein, "stringent conditions" may be any of low, moderate, and high stringent conditions. The "low stringent condition" refers to a condition with, for example, 5 × SSC, 5 × Denhardt's solution, 0.5% SDS, 50% formamide, and 32°C. The "moderate stringent condition" refers to condition with, for example, 5 × SSC, 5 × Denhardt's solution, 0.5% SDS, 50% formamide, and 42°C. The "high stringent condition" refers to a condition with, for example, 5 × SSC, 5 × Denhardt's solution, 0.5% SDS, 50% formamide, and 50°C. Under these conditions, highly homologous DNA is expected to be obtained more efficiently at a higher temperature. While multiple factors including the temperature, the probe concentration, the probe length, the ionic strength, the time, the salt concentration, and the like are expected to be involved in the determination of the hybridization stringency, those skilled in the art can appropriately select these factors to achieve similar stringency.

Examples of a hybridizable polynucleotide include polynucleotides having, for example, 70% or more, 80% or more, 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more identity to a control polynucleotide sequence, as calculated by a homology search software such as FASTA and BLAST using default parameters. In general, the higher the percentage of homology above, the more preferable.

The identity or the homology of the amino acid sequence or the polynucleotide sequence can be determined using the BLAST algorithm of Karlin and Altschul (Proc. Natl. Acad. Sci. USA 87: 2264-2268, 1990; Proc. Natl. Acad. Sci USA 90: 5873, 1993). The programs derived from the BLAST algorithm, so-called BLASTN and BLASTX, have been developed (Altschul SF, et al: J Mol Biol 215: 403, 1990). If BLASTN is used to analyze the nucleotide sequence, the parameters are available with, for example, as follows: Expect threshold = 100, Word size = 12. Furthermore, if BLASTX is used to analyze the amino acid sequence, the parameters are available with, for example, as follows: Expect threshold = 50, Word size = 3. If BLAST and Gapped BLAST programs are used, the default parameters of each program are preferably used, which may appropriately be altered within a range known to those skilled in the art.

### 2. Cross-reactivity with neutralizing antibodies

The AAV vector according to the present invention does not cross-react at least with a neutralizing antibody against AAV serotype 2 (AAV2) present in the serum, and possibly further with neutralizing antibodies against AAV serotype 1 (AAV1) and/or AAV serotype 8 (AAV8), or other wild-type AAV.

According to the present invention, a neutralizing antibody refers to an antibody that binds to an antigen when said antigen has an activity such as toxicity or infectivity to a living body, thereby reducing or eliminating said activity. According to the present invention, the term "neutralization" in the context of activity of an antibody means, for example, that an anti-AAV neutralizing antibody (e.g., IgG, IgM, IgA) in a serum binds to the AAV vector, i.e., the antigen, resulting in reduction or elimination of the gene transfer capacity of said AAV vector. This neutralization reaction comprises a series of complement reactions including binding between the antigen (e.g., the AAV vector) and the antibody, binding between the Fc portion of the antibody and the first complement component (C 1), and the like, which results in reduction or elimination of the infectivity (gene transfer capacity) of the AAV vector to the target cell. Moreover, according to the present invention, a neutralizing antibody is expected to be associated with a serum component, specifically, expected to contain a component (e.g., a complement) bound to an antibody and directly involved in the neutralization reaction such as inactivation or removal of the antigen.

According to the present invention, cross reactivity of a neutralizing antibody refers to a reaction where the neutralizing antibody binds to a target substance (e.g., AAV3) different from the originally targeted antigen (e.g., AAV2). Moreover, according to the present invention, "not cross-reactive with a neutralizing antibody" means that a neutralizing antibody does not function sufficiently in binding (preferably does not provide any detectable binding) to a target substance other than the originally targeted antigen, or means that, even if the binding is detectable, the amount bound to the target substance (mole ratio or mass ratio) relative to the amount bound to the originally targeted antigen is significantly reduced. Such a reduction in the amount bound to the target substance may be, for example, several % (about 5%), about 10%, about 15%, about 20%, about 25%, about 30%, about 35%, about 40%, about 45%, about 50%, or about 60% relative to the amount of binding to the originally targeted antigen. Such a percentage can be determined using a known method such as a method of measuring an amount of the protein binding to the antibody. Alternatively, cross reactivity of a neutralizing antibody can also be measured and compared indirectly using a protein coded in the recombinant virus (e.g., a reporter gene).

A neutralizing antibody according to the present invention is preferably derived from a mammal, more preferably from a primate, and most preferably from a human. Unless otherwise indicated, the neutralizing antibody used in the present invention refers to a neutralizing antibody against AAV2. Usually, human is highly likely to have antibodies against AAV2, but majority of them do not have neutralizing capacity and some reports say that the proportion of the antibodies having neutralizing capacity is 18-32% (Chirmule N. et al., Gene Ther 6: 1574-1583, 1999; Moskalenko M, et al., J Virol 74: 1761-1766, 2000). Furthermore, most of the human sera containing neutralizing antibodies against AAV2 are known to contain neutralizing antibodies against AAV3A and AAV3B (Fu H et al., Hum Gene Ther Clin Dev 2017; 28: 187-196, Ling CJ et al., Integr Med 2015; 13: 341-346). This is also suggested by a relatively high amino acid identity between the AAV2 VP1 protein (SEQ ID NO:1) and the AAV3A or AAV3B VP1 protein (SEQ ID NO:2 or 3) (specifically, about 87-88%).

Furthermore, a neutralizing antibody used in the present invention may include multiple isotypes (e.g., IgG, IgM, IgA, etc.). Therefore, a neutralizing antibody against AAV2 according to the present invention needs to be investigated for its performance including an antibody concentration or an antibody titer in advance. Procedures for such investigation are known in the art. For example, the procedure described in Ito et al. (Ito et al., Ann Clin Biochem 46: 508-510, 2009) can be employed. Specifically, a titer of a neutralizing antibody (antibody titer) can be analyzed by assessing the potency of inhibiting the introduction of a AAV2 vector containing a reporter gene such as β-galactosidase, luciferase, etc. into HEK293 cells (Ito et al., *op. cit.*)*.*

The neutralizing antibody used in the present invention has an antibody titer of 1:4-1:640, preferably 1:16-1:254, and more preferably 1:32-1:128. For the specific method for measuring the antibody titer according to the present invention, see the descriptions under the sections "(e) Verification of cross-reactivity with neutralizing antibodies in serum", "(3) Measurement of antibody titers of neutralizing antibodies" and the other in EXAMPLES.

In addition, as a specific procedure of an assay for verifying the cross-reactivity with a neutralizing antibody, methods described in Li C, et al., Gene Ther 19: 288-294, 2012, Meliani A, et al., Hum Gene Ther Methos 26: 45-53, 2015, etc. can also be employed.

The AAV vector according to the present invention is less susceptible to the neutralizing antibody in the serum as compared to a wild type. Specifically, even after being treated with a neutralizing antibody in a serum, the virus vector according to the present invention (e.g., one including a capsid protein having the amino acid sequence represented by SEQ ID NO:4) maintains at least 60%, preferably 70% or more, more preferably 75% or more, still more preferably 80% or more, 85% or more, 90% or more, or 95% or more of the capacity of transferring a gene into target cells. Here, for comparison of the gene transfer capacity, a known assay such as reporter assay, *in situ* hybridization, or radioisotope labeling can be employed. On the other hand, a wild-type virus vector (e.g., one including a capsid protein having the amino acid sequence represented by SEQ ID NO:2 or 3) similarly treated with the neutralizing antibody in the serum, may maintain less than about 60%, less than about 50%, less than about 40%, less than about 35%, or less than about 30% of the capacity of transferring a gene into target cells.

In other words, the virus vector according to the present invention is capable of transferring a gene into target cells in an amount 1.2 times or more, preferably 1.5 times or more, more preferably 1.75 times or more, and still more preferably twice or more as compared with the amount of a gene transferred with a wild-type virus vector, after being treated with a neutralizing antibody in a serum.

According to the present invention, a neutralizing antibody against, for example, AAV2 is known to have a neutralizing activity against AAV3 or others as well due to cross-reactivity (Fu H et al., Hum Gene Ther Clin Dev 2017; 28: 187-196, Ling CJ et al., Integr Med 2015; 13: 341-346, Mimuro J, et al., J Med Virol 86: 1990-1997, 2014). The identity between the amino acid sequences of AAV2 capsid protein VP1 and AAV3A VP1 is calculated to be 87%, and the identity between the amino acid sequences of AAV2 VP1 and AAV3B VP1 is calculated to be 88% (which are results obtained by using the website of Blastp (https://blast.ncbi.nlm.nih.gov/Blast.cgi)). Furthermore, studies relating to epitope mapping of an anti-AAV2 neutralizing antibody is described in Moskalenko, M. et al. (J Virol 74: 1761-1766, 2000).

The followings are known as extracellular receptors involved in infection of cells with AAV (Summerfold et al., Mol Ther 24: 2016; Pillay et al., Nature 530: 108-112, 2016).

### Primary receptors

- AAV2, 3, 6: Heparan sulfate proteoglycan
- AAV9: Terminal N-linked galactose
- AAV1, 4, 5, 6: Specific N-linked or O-linked sialic acid moiety Secondary receptors
- AAV2: Fibroblast growth factor receptor and integrin
- AAV2, 3: Hepatocyte growth factor receptor (c-Met)
- AAV5: Platelet-derived growth factor (which may be modified with sialic acid)

In light of the disclosure of the present application and the findings relating to the above-described receptors, a recombinant vector which is even less susceptible to inhibition caused by a neutralizing antibody against AAV2 or the like in a living body may also be designed, screened, and obtained based on the recombinant virus vector according to the present invention.

### 3. Genes contained in virus vector of the present invention

In one embodiment, the rAAV vector of the present invention preferably comprises a polynucleotide containing a liver-specific promoter and a gene of interest operably linked to said promoter (specifically, such a polynucleotide is packaged). The promoter used in the present invention may be a promoter specific to cells in the liver, for example, a promoter specific to hepatic parenchymal cells, hepatic non-parenchymal cells (such as hepatic stellate cells) or the like. Examples of such a promoter sequence specifically include, but are not limited to, an ApoE promoter, an antitrypsin promoter, a cKit promoter, a promoter for a liver-specific transcription factor (e.g., HNF-1, HNF-2, HNF-3, HNF-6, C/ERP, and DBP), a thyroxine-binding globulin (TBG) promoter (Ran FA, et al., Nature 520 (7546): 186-91, 2015), a promoter for other liver-specific protein (albumin, etc.), and a synthetic promoter obtained by combining these promoters. Furthermore, these promoters can be combined with a known enhancer, preferably a liver-specific enhancer. Examples of such an enhancer include an ApoE enhancer. These promoters and enhancers can be used alone or in any combination. Alternatively, a synthetic promoter that utilizes the above-described liver-specific promoter and enhancer can also be used. The rAAV vector of the present invention may preferably contain a liver-specific promoter, and more preferably a hepatocyte (hepatic parenchymal cell)-specific promoter, including an ApoE promoter, an antitrypsin promoter, a TBG promoter, or an HCRhAAT promoter which is a known synthetic promoter (for example, the promoter having the sequence of nucleotides 397-1046 of SEQ ID NO: 12).

Further, the liver-specific promoter (or enhancer) used in the present invention may also be a polynucleotide containing a sequence which has one or more (e.g., 1-100, 1-50, 1-40, 1-30, 1-25, 1-20, 1-15, 1-10, 1-9 (one to several), 1-8, 1-7, 1-6, 1-5, 1-4, 1-3, 1-2, 1, etc.) nucleotides deleted, substituted, inserted, and/or added in the polynucleotide sequence of the above-described promoter, and which can serve as a liver-specific promoter. Herein, "to serve as a liver-specific promoter" means that when, for example, liver-derived cells and non-liver-derived cells are compared in a reporter assay, the non-liver-derived cells express the reporter at around the threshold level of detection or express the reporter at a level about 25% or less, about 20% or less, about 15% or less, or about 10% or less as compared with a level at which the liver-derived cells express the reporter. In addition, a polynucleotide having the above-described deletion, substitution, insertion, and/or addition means to have a specific activity of 50%, 60%, 70%, 80%, 90% or more relative to that of the original promoter sequence. The smaller the number of the above-described mutations, the better.

Examples of a disease targeted by a treatment using the AAV vector of the present invention include ornithine transcarbamylase deficiency, hemophilia, acute intermittent porphyria, Wilson's disease, phenylketonuria, and familial hypercholesterolemia, which involve genomic disorders of hepatocytes.

To perform the above-described therapy, the recombinant virus vector used in the present invention can contain any of a variety of therapeutic genes (polynucleotides). Examples of a protein encoded by such a therapeutic gene include, but are not limited to, proteins such as ornithine transcarbamylase (OTC), carbamoyl-phosphate synthase I (CPS1), coagulation factor VIII (FVIII), coagulation factor IX (FIX), hepatocyte growth factor (HGF), and hepatocyte growth factor receptor (c-Kit).

Ornithine transcarbamylase deficiency is a disorder involving the ornithine transcarbamylase gene and is caused by dysfunction of the urea cycle in the liver. The liver of a living body produces harmless urea from ammonia toxic to a living body, and this pathway is called the urea cycle. Examples of enzymes involved in this urea cycle include ornithine transcarbamylase (OTC), carbamoyl-phosphate synthase I (CPS1), argininosuccinate synthase (ASS), argininosuccinate lyase (ASL), arginase I (ARG1), N-acetylglutamate synthase (NAGS), and omithine/citrulline antiporter (ORNT1). Genetic defects in these enzymes result in urea cycle disorders. Among these urea cycle disorders, OTC deficiency is the most common disorder.

The mature form of OTC consists of a trimer of approximately 36 kda (in case of human) protein (the amino acid sequence represented by SEQ ID NO:11) encoded on the X chromosome. Specifically, OTC converts ornithine into citrulline in the urea cycle ((3) in the figure below).

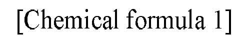

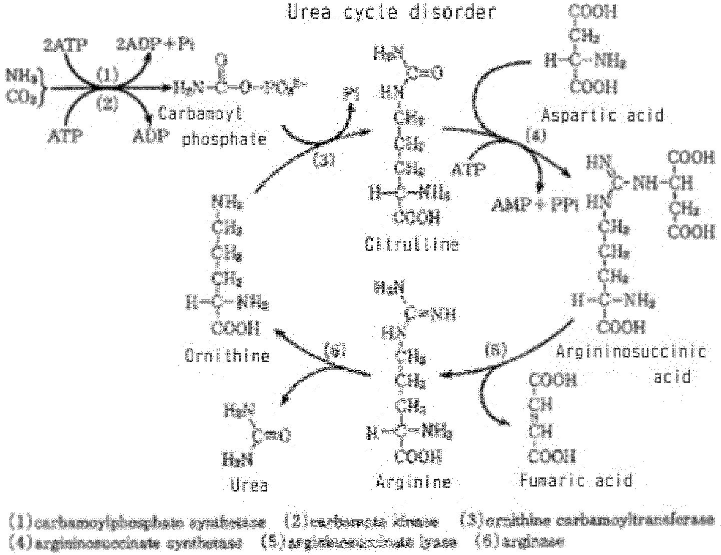

### (From "Iwanami Dictionary of Biology, 4th Edition," Iwanami Shoten)

The OTC used in the present invention is not particularly limited as long as it is an enzyme that can convert ornithine to citrulline, and examples thereof preferably include those derived from mammals, and more preferably those derived from human. For example, a protein having the amino acid sequence represented by SEQ ID NO:11 can be used as the OTC used in the present invention. Alternatively, mutants that retain, as the ornithine transcarbamylase activity, a specific activity of preferably 40% or more, 50% or more, 60% or more, 70% or more, 80% or more, 90% or more, 95% or more, or 99% or more as compared with the wild type can also be used. Examples of such mutants include, but are not limited to, those having an amino acid sequence with 60% or more, 70% or more, 80% or more, 90% or more, 95% or more, 96% or more, 97% or more, 98% or more, or 99% or more identity to the amino acid sequence represented by SEQ ID NO:11.

As the method for measuring OTC, for example, a measurement method utilizing the enzyme activity (Pierson et al. (1977) J. Biol. Chem. 252, 6464-6469) and an immunological measurement method (WO 2006/073073) are known in the art. In the present invention, any measurement method known in the art can be used to measure OTC. Furthermore, reduction in the concentration of ammonia by administration of the vector of the present invention can be confirmed by measuring the concentration of ammonia in blood or culture medium by a means known in the art.

The therapeutic gene used in the present invention may be, but is not limited to, a gene for inhibiting the function of a target (antisense nucleotides, CAS9, etc.).When used herein, antisense nucleotides refer to a polynucleotide for altering (e.g., disrupting or reducing) a function of a targeted endogenous gene, or a polynucleotide for altering (e.g., reducing) the expression level of an endogenous protein. Examples of such a polynucleotide include, but are not limited to, an antisense molecule, a ribozyme, interfering RNA (iRNA), microRNA (miRNA), and sgRNA. Methods for preparing and using a double-stranded RNA (dsRNA, siRNA, shRNA, or miRNA) are known from many literature (see, JP-A-2002-516062; US 2002/086356A; Nature Genetics, 24(2), 180-183, 2000 Feb., etc.). Furthermore, the recombinant virus vector used in the present invention may contain one or more therapeutic genes.

### 4. Pharmaceutical composition

In another embodiment of the present invention, a pharmaceutical composition containing the rAAV vector (rAAV virion) of the present invention is provided. The pharmaceutical composition containing the rAAV vector of the present invention (hereinafter, referred to as the pharmaceutical composition of the present invention) can be used to transfer a therapeutic gene into liver cells of a subject in a highly efficient manner. Thus, a pharmaceutical composition which can treat a disease of interest through expression of the introduced therapeutic gene is provided. The pharmaceutical composition of the present invention comprises the rAAV vector containing such a therapeutic gene. Examples of such a therapeutic gene include, but are not limited to, the above-mentioned genome editing means and genome repairing means.

In one embodiment, the rAAV vector of the present invention preferably contains a promoter specific to liver cells and a gene operably linked to said promoter. The rAAV vector of the present invention can contain a gene effective for a treatment of hemophilia or others so that such a gene can be transferred into liver cells, preferably into hepatic parenchymal cells.

The pharmaceutical composition of the present invention can be in use, for example, administered orally, parenterally (intravenously), muscularly, through oral mucosa, rectally, intravaginally, transdermally, intranasally, or via inhalation, and parenteral administration is preferred and intravenous administration is more preferred. While an active ingredient of the pharmaceutical composition of the present invention may be formulated alone or in combination, a pharmaceutically acceptable carrier or additive for a formulation can be added thereto to provide a formulation form. In this case, the content of the active ingredient of the present invention in the formulation may be, for example, 0.1-99.9% by weight.

While an active ingredient of the pharmaceutical composition of the present invention may be formulated alone or in combination, a pharmaceutically acceptable carrier or additive for formulation can be added thereto to provide a formulation form. In this case, the active ingredient of the present invention is contained in the formulation in an amount that gives, for example, a titer of 10⁵-10¹⁶ vg/mL (900 fg/mL-90 mg/mL), a titer of 10⁶-10¹⁵ vg/mL (9.0 pg/mL-9.0 mg/mL), a titer of 10⁷-10¹⁴ vg/mL (90 pg/mL-900 µg/mL), a titer of 10⁸-10¹³ vg/mL (900 pg/mL-90 µg/mL), a titer of 10⁹-10¹² vg/mL (9 ng/mL-9 µg/mL), or a titer of 10¹⁰-10¹¹ vg/mL (90 ng/mL-900 ng/mL). Moreover, a pharmaceutically acceptable carrier and/or additive may be used, including, for example, an excipient, a disintegrant, a disintegration aid, a binder, a lubricant, a coating agent, a dye, a diluent, a dissolution agent, a dissolution aid, a tonicity-adjusting agent, a pH regulator, and a stabilizer.

Examples of the formulation suitable for parenteral administration include an injection and a suppository. For parenteral administration, a solution obtained by dissolving the active ingredient of the present invention in either sesame oil or peanut oil or in an aqueous propylene glycol solution or an aqueous sorbitol solution can be used. The aqueous solution should first be appropriately buffered as needed (preferably, pH 8 or higher) so that the liquid diluent is isotonic. For example, physiological saline can be used as such a liquid diluent. The prepared aqueous solution is suitable for an intravenous injection whereas the oily solutions are suitable for an intraarticular injection, an intramuscular injection, and a subcutaneous injection. All of these solutions can be produced readily under aseptic conditions by standard pharmaceutical preparation techniques well known to those skilled in the art. Furthermore, the active ingredient of the present invention can also be applied topically to the skin or the like. In this case, the topical application is desirably carried out in a form of cream, gel, paste or an ointment in accordance with a standard pharmaceutical practice.

Examples of the formulation suitable for oral administration include powder, a tablet, a capsule, fine granules, granules, a liquid agent, and a syrup. For oral administration, any of various excipients such as microcrystalline cellulose, sodium citrate, calcium carbonate, dipotassium phosphate or glycine may be used together with a starch (preferably a starch of corn, potato, or tapioca), any of various disintegrants such as alginic acid or a certain multiple salt of silicic acid, and/or a granulation binder such as polyvinylpyrrolidone, sucrose, gelatin, or gum arabic.

The dose of the pharmaceutical composition of the present invention is not particularly limited, and a suitable dose can be selected depending on various conditions including the type of the disease, age and symptoms of the patient, the administration route, the therapeutic goal, the presence of medicine used in combination therewith, and the like. A daily dose of the pharmaceutical composition of the present invention is, for example, but not limited to, 1-5,000 mg, preferably 10-1,000 mg, per adult (e.g., body weight 60 kg). Such a dose may be given in two to four doses a day. In terms of vg (vector genome) as the dose unit, the dose can be selected from, for example, but is not limited to, a range of 10⁶-10¹⁴ vg, preferably 10⁸-10¹³ vg, and more preferably 10⁹-10¹² vg per 1 kg body weight.

### 5. Administration of virus vector of the present invention

The virus vector of the present invention can be administered to a subject preferably by peripheral administration for further safer and easier administration. Herein, peripheral administration refers to administration routes generally understood as peripheral administrations by those skilled in the art, including intravenous administration, intraarterial administration, intraperitoneal administration, intracardiac administration, and intramuscular administration.

When administered to a subject, the virus vector of the present invention infects liver cells, and provides the above-described genome editing means delivered by the virus in the infected cells, resulting in genome editing. The virus vector of the present invention preferably infects hepatic parenchymal cells to effect genome editing. The virus vector of the present invention preferably causes 70% or more, 80% or more, 85% or more, 90% or more, 91% or more, 92% or more, 93% or more, 94% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, 99.9% or more, or 100% of the hepatic parenchymal cells in the liver of the subject administered with said virus vector to be subjected to genome editing.

### 6. Kit for preparing rAAV vector of the present invention

In another embodiment of the present invention, the present invention provides a kit for preparing the rAAV of the present invention. Such a kit comprises, for example, (a) a first polynucleotide for expressing a capsid protein VP1, etc., and (b) a second polynucleotide to be packaged into the rAAV vector. For example, the first polynucleotide comprises a polynucleotide coding for the amino acids represented by SEQ ID NO:4, and the second polynucleotide may or may not contain a therapeutic gene of interest. Preferably, it can contain various restriction enzyme cleavage sites for incorporating such a therapeutic gene of interest.

The kit for preparing the rAAV virion of the present invention may further include any component described herein (e.g., AdV helper, etc.). In addition, the kit of the present invention may further include instructions describing the protocol for preparing the rAAV virion by using the kit of the present invention.

7. Other terms used in this specification

The meaning of each term used herein is as follows. Terms are intended to have the meanings within the scope commonly understood by those skilled in the art, unless otherwise particularly explained herein.

As used herein, unless otherwise stated, the terms "virus vector", "virus virion", and "virus particle" are used interchangeably. In addition, the term "adeno-associated virus vector" includes recombinant adeno-associated viruses.

As used herein, the term "polynucleotide" is used interchangeably with "nucleic acids", "gene" or "nucleic acid molecule", and is intended to refer to a polymer of nucleotides. As used herein, the term "nucleotide sequence" is used interchangeably with "nucleic acid sequence" or "base sequence" and is represented by a sequence of deoxyribonucleotides (abbreviated as A, G, C, and T). For example, a "polynucleotide comprising the nucleotide sequence represented by SEQ ID NO: 1 or a fragment thereof' is intended to refer to a polynucleotide comprising the sequence represented by the respective deoxynucleotides A, G, C and/or T of SEQ ID NO: 1, or a fragment thereof.

"Virus genome" and "polynucleotide" according to the present invention may each exist in a form of DNA (e.g., cDNA or genomic DNA) or, in some cases, in a form of RNA (e.g., mRNA). The virus genome and the polynucleotide as used herein may each be a double-stranded or single-stranded DNA. The single-stranded DNA or RNA may be a coding strand (also known as a sense strand) or a non-coding strand (also known as an antisense strand).

Unless otherwise specified, when a promoter, a gene of interest, a polyadenylation signal, and else encoded by the rAAV genome are described herein with respect to their locations in the gene, the strand itself is described if the rAAV genome is a sense strand and its complementary strand is described if it is an antisense strand. Herein, "r" representing "recombination" may be omitted when it is apparent from the context.

As used herein, the terms "protein" and "polypeptide" are used interchangeably and intended to refer to a polymer of amino acids. The polypeptide as used herein is represented in accordance with a conventional peptide designation, in which the N-terminal (amino terminal) is shown on the left and the C-terminal (carboxyl terminal) on the right. Partial peptides of the polypeptide of the present invention (which, may be simply referred to herein as "partial peptides of the present invention") includes partial peptides of the polypeptide of the present invention described above, and preferably has the same properties as said polypeptide of the present invention.

As used herein, the term "plasmid" refers to any of various known gene elements, for example, a plasmid, a phage, a transposon, a cosmid, a chromosome, etc. The plasmid can replicate in a particular host and transport gene sequences cell to cell. As used herein, a plasmid contains various known nucleotides (DNA, RNA, PNA, and a mixture thereof) and may be a single strand or a double strand, preferably a double strand. For example, as used herein, unless otherwise specified, the term "rAAV vector plasmid" is intended to include a double strand formed of a rAAV vector genome and its complementary strand. The plasmid used in the present invention may be either linear or circular.

As use herein, the term "packaging" refers to events including preparation of a single-stranded virus genome, assembly of a coat (capsid) protein, enclosure of the virus genome within the capsid (encapsidation), and the like. When an appropriate plasmid vector (normally, a plurality of plasmids) is introduced into a cell line that allows packaging under appropriate conditions, recombinant virus particles (i.e., virus virions, virus vectors) are constructed and secreted into the culture.

For terms that are not particularly described herein are intended to have the meanings within the scope commonly understood by those skilled in the art.

### EXAMPLES

Hereinafter, the present invention will be described more specifically by means of examples; however, the scope of the present invention should not be limited to the following examples.

### A. Materials and methods

### (a) AAV vectors

Five AAV vectors (AAV2, AAV3B, AAV.GT5, AAV8, and AAV-LK03) were used.

AAV.GT5 contains three amino acid substitutions in coat proteins VP1 of AAV3A and 3B, namely the amino acid sequence in which serine (S) at position 472 is substituted with alanine (A), serine (S) at position 587 is substituted with alanine (A), and asparagine (N) at position 706 is substituted with alanine (A). Meanwhile, AAV.M1 contains one amino acid substitution in the coat proteins VP1 of AAV3A and 3B, namely the amino acid sequence in which serine (S) at position 587 is substituted with alanine (A). AAV.M2 contains two amino acid substitutions in the coat proteins VP1 of AAV3A and 3B, namely the amino acid sequence in which serine (S) at position 472 is substituted with alanine (A) and serine (S) at position 587 is substituted with alanine (A).

Three types of genes were used as genes encoding proteins to be expressed in each AAV vector.

For a green fluorescent protein (AcGFP), an expression cassette composed of a cytomegalovirus (CMV) promoter, cDNA of green fluorescence protein (AcGFP), and SV40 poly(A) was inserted between the inverted terminal repeats (ITRs) of AAV3A to prepare four vectors, namely, AAV2-CMV-AcGFP, AAV3B-CMV-AcGFP, AAV.GT5-CMV-AcGFP, and AAV8-CMV-AcGFP. For a luciferase, a sequence encoding the luciferase instead of AcGFP was inserted to prepare four vectors, namely, AAV2-CMV-Luciferase, AAV3B-CMV-Luciferase, AAV.GT5-CMV-Luciferase, and AAV-LK03-CMV-Luciferase. For the OTC, an expression cassette composed of an enhancer of the liver regulatory region of ApoE/C1 gene and a human antitrypsin promoter (HCRhAAT), cDNA of OTC, and SV40 poly(A) was inserted between the inverted terminal repeats (ITRs) of AAV3A to prepare two vectors, namely AAV.GT5-HCRhAAT-OTC and AAV8-HCRhAAT-OTC.
AAV2: GenBank Accession #NC_001401.2 (whole genome sequence)
   (The amino acid sequence of AAV2 capsid protein VP1 is represented by SEQ ID NO:1.)
AAV3A: GenBank Accession #U48704 (genome sequence)
   (The amino acid sequence of AAV3A capsid protein VP1 is represented by SEQ ID NO:2.)
AAV3B: GenBank Accession #AF028705.1 (genome sequence)
   (The amino acid sequence of AAV3B capsid protein VP1 is represented by SEQ ID NO:3.)
AAV.GT5: (prepared in this application)
   (The amino acid sequence of capsid protein VP1 is represented by SEQ ID NO:4.)
AAV8: GenBank Accession #NC_006261 (genome sequence)
   (The amino acid sequence of AAV8 capsid protein VP1 is represented by SEQ ID NO:5.)
AAV-LK03: (The amino acid sequence of capsid protein VP1 is represented by SEQ ID NO:9.) (Lisowski L. et al., Nature, 2014, February 20; 506 (7488): 382-386)
AAV.M1: (prepared in this application)
   (The amino acid sequence of capsid protein VP1 is represented by SEQ ID NO:17.)
AAV.M2: (prepared in this application)
   (The amino acid sequence of capsid protein VP1 is represented by SEQ ID NO:18.)

### (b) Cell culture

### 1) HEK 293 cells

HEK293 cells were seeded at 2 × 10⁴ or 5 × 10⁴ cells/well and cultured using a 10% fetal calf serum (FCS)-DMEM/F12 medium (Thermo Fisher Scientific) in 5% CO₂ at 37°C.

### 2) HepG2 cells

HepG2 cells were seeded at 2 × 10⁴ or 5 × 10⁴ cells/well and cultured using a 10% FCS-DMEM low glucose medium (Thermo Fisher Scientific) in 5% CO₂ at 37°C.

### 3) PXB cells (PhoenixBio Co., Ltd.)

PXB cells were collected from a PXB mouse liver and composed of 90% or more human hepatocytes. The cells were seeded at 7 × 10⁴ (96 well plate) or 7.4 × 10⁵ (12 well plate) cells/well and cultured using a specialized dHCGM medium (PhoenixBio Co., Ltd.) in 5% CO₂ at 37°C.

The activity of a neutralizing antibody against AAV2 was measured by the method described in Melian A et al., HUMAN GENE THERAPY METHODS, 26: 45-52, 2015 and sera from ten persons which showed neutralizing antibody titers of 1:32-1:128 or 1:128 or higher were used.

### (c) Vector infection

To each cell culture, the AAV vectors for expressing GFP (AAV2-CMV-AcGFP, AAV8-CMV-AcGFP, AAV3B-CMV-AcGFP, and AAV.GT5-CMV-AcGFP), described in (a) were each added at 2 × 10³-1 × 10⁴ vg/cell, the AAV vectors for expressing Luciferase (AAV2-CMV-Luciferase, AAV3B-CMV-Luciferase, AAV.GT5-CMV-Luciferase, and AAV-LK03-CMV-Luciferase) were each added at 400 or 800 vg/cell, and the vectors for expressing OTC (AAV.GT5-HCRhAAT-OTC, and AAV8-HCRhAAT-OTC) were each added at 4 × 10⁴ vg/cell, and then cells were cultured for 1-10 days.

### (d) Evaluation of GFP or luciferase expression

To measure the GFP expression, the fluorescence intensities of GFP expressed by the variety of AAV vectors were measured and compared using a plate reader (Biotech Japan Corporation). In addition, images of representative fields of view of the GFP-expressing cells were taken using a fluorescence microscope (Olympus IX83).

To measure the activity of luciferase, a reagent containing a cell lysis buffer, luciferin, etc. (Bright-Glo^{™} Luciferase Assay System, Promega) was added to each cell, and the luminescence intensity was measured and compared using a plate reader (Biotech Japan Corporation).

### (e) Verification of cross-reactivity with neutralizing antibodies in serum

Luciferase was used as a reporter to confirm resistance to the neutralizing antibodies. AAV2, AAV3B, and AAV-LK03 (known, neutralizing antibody-resistant, modified AAVs: Perocheau, D.P. et al., (2018), HUMAN GENE THERAPY, 30,1, 79-87) were used as comparison controls.

The series of two-fold dilution for the 10 types of sera collected from healthy subjects were prepared with FCS, which ranged from 1:2 to 1:128. The respective four types of AAVs (AAV.GT5-CMV-Luciferase, AAV-LK03-CMV-Luciferase, AAV3B-CMV-Luciferase, and AAV2-CMV-Luciferase) were mixed with these sera at a ratio of 1:1, allowed to react at 37°C for 1 hour, and then administered to HEK 293 cells. After 24 hours, luminescent activity of luciferase was measured using a Luciferase Assay Kit. The relative luminescence activity of each AAV was expressed in %, given that the value of the control, i.e., AAV mixed with FCS instead of the sample serum was 100%. The maximum dilution rate that suppressed the luciferase activity to 50% or below was defined as the neutralizing antibody titer.

### (f) Preparation of ornithine carbamyltransferase (OTC) expression vector

AAV vectors each incorporating human ornithine carbamyltransferase cDNA (SEQ ID NO: 10) were prepared by using conventional genetic recombination methods, etc.

### (g) Confirmation of OTC expression

PXB cells were seeded at 7.4 × 10⁵/well in three 12-well plates and cultured in a special dHCGM medium (PhoenixBio Co., Ltd.) in 5% CO₂ at 37°C. After 6 days of cultivation, AAV.GT5-HCR-hAAT-OTC and AAV8-HCRhAAT-OTC were administered at 4 × 10⁴ vg/cell to 4 wells per group. To confirm the endogenous OTC level, 4 wells of non-administered cells were used as controls. The medium was changed 3 and 7 days after the administration while the cells were cultured in 5% CO₂ at 37°C. The culture was terminated 10 days after the administration to measure OTC mRNA, OTC protein, and OTC enzyme activities.

### (Measurement of OTC mRNA)

Total RNAs were purified from the PXB cells (n=4) administered with AAV.GT5-HCRhAAT-OTC and AAV8-HCRhAAT-OTC using RNeasy Mini Kit (QIAGEN). cDNA was synthesized from 100 ng of the total RNA, and High Capacity cDNA Reverse Transcription Kit with RNase Inhibitor (Applied Biosystems) was used for reverse transcription reaction. The cDNA samples were diluted 6-fold with nuclease-free water, and the Ct values were measured by SYBR Green method for real time PCR (Applied Biosystems). The primers with the optimized OTC sequence were prepared.
OTC Forward: ACCGGCGAAGAGATCAAGTA (SEQ ID NO: 13)
OTC Reverse: ATCATGCCCAGAGACTTTCC (SEQ ID NO: 14)
For normalization, the following sequences were also used in the detection of GAPDH.
GAPDH Forward: AATTCCATGGCACCGTCAAG (SEQ ID NO: 15)
GAPDH Reverse: ATCGCCCCACTTGATTTTGG (SEQ ID NO: 16)

Comparative Ct method (ΔΔCt method) was employed to analyze each mRNA expression level. The relative OTC levels of the AAV.GT5-HCRhAAT-OTC-administered group were calculated using GAPDH gene as an internal standard, given that the respective expression levels of the AAV8-HCRhAAT-OTC-administered group were 1.

### (Measurement of OTC protein)

The PXB cells at the end of culture were rinsed with PBS and lysed by adding 100 µl/well of RIPA Buffer + protease inhibitor. After collecting the supernatant by centrifugation, Laemmli Buffer + βME was added and the resultant was heated to reduce proteins.

The proteins were electrophoresed with 4-15% polyacrylamide gel and Tris/glycine/SDS buffer, transferred to PVDF membrane, blocked, and then allowed to react with OTC antibody (1:2,000, SantaCruz) at 4°C overnight. After washing 6 times with 0.1% PBST, the proteins were allowed to react with HRP-anti-mouse-IgG (1:5,000, GE Healthcare) for 60 minutes at room temperature. After washing 6 times with 0.1% PBST, ECL Prime Western Blotting Detection Reagent (GE Healthcare) was used to allow for chemiluminescence, which was detected with Amersham Imager 600, and the Volume values of the bands were determined with the built-in analysis software. Similarly, the housekeeping protein levels were measured using GAPDH antibody (1:10000, Abcam) and compared with the corrected OTC volume value/GAPDH volume value.

### (Measurement of OTC activity)

PXB cells were scraped from the dish and centrifuged (1,000 rpm, 3 minutes, room temperature) to remove the medium. The cells were further washed twice with PBS. Mitochondrial lysis buffer (0.5% Triton X-100 [v/v] in dH₂O, 10 mM HEPES, 2 mM DTT [pH 7.4]) was added and the cells were homogenized on ice. Centrifugation (20,630 G, 20 minutes, 4°C) was performed, and the supernatants were collected to determine the amounts of the proteins. The protein amounts of 5-10 µg were adjusted to the volume of 10 µl, and the reaction solution (5 mM ornithine, 15 mM carbamyl phosphate, 270 mM triethanolamine) was added thereto to the total volume of 700 µl. The mixtures were incubated at 37°C for 60 minutes, and 270 µL of Stop solution (0.5% antipyrine/50% sulfuric acid (w/v): 0.8% 2,3-butanedion monoxime: distilled water [1:1:1.5]) was added thereto. The mixtures were boiled while shielding light for 15 minutes. Two hundred microliters of the resultants were placed in a 96-well plate to determine the absorbance values at a wavelength of 490 nm. The enzyme activity was expressed as µmοl of citrulline produced/mg of liver protein/hr.

### B. Results

### (1) Preparation of modified AAV.GT5

DNAs containing the fused Rep sequences of the AAVs AAV3A and AAV3B, and the VP sequence of AAV3B were artificially synthesized. At the step, genetic engineering was performed to provide S472A, S587A and N706A mutations in the AAV3B VP1, thereby obtaining the modified adeno-associated virus AAV.GT5.

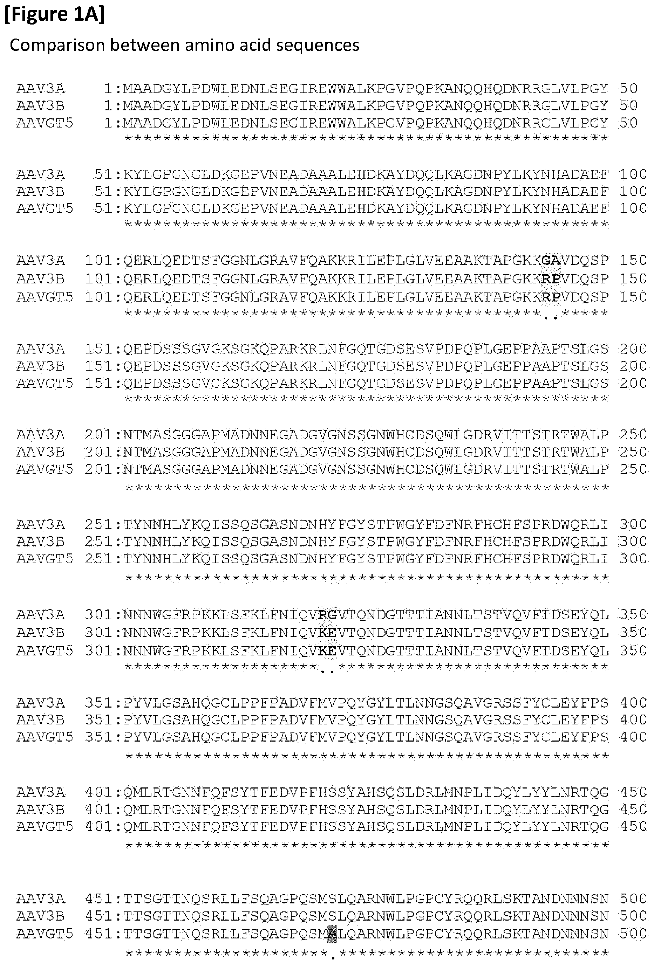

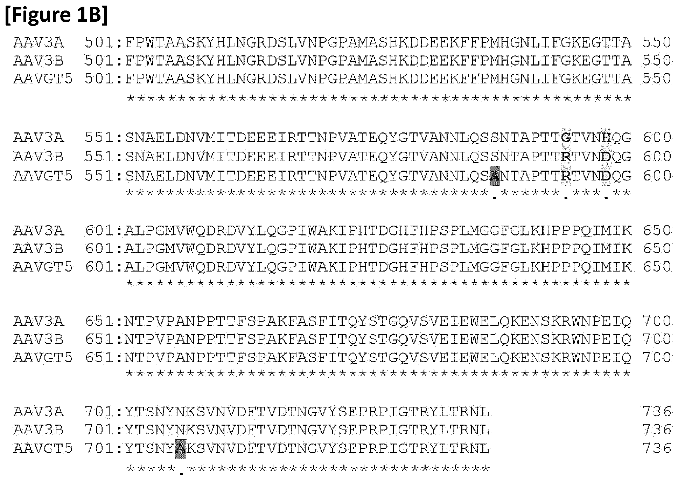

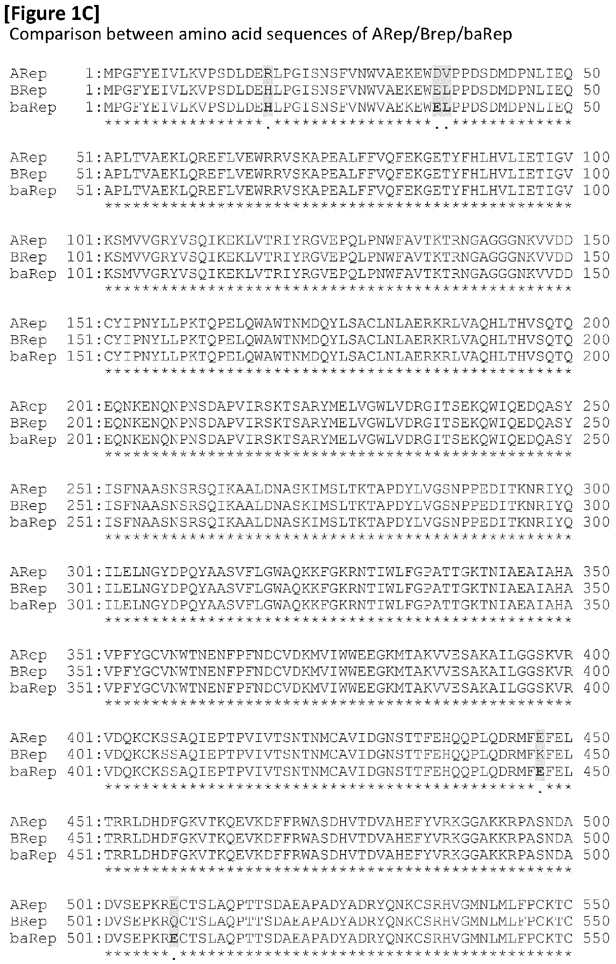

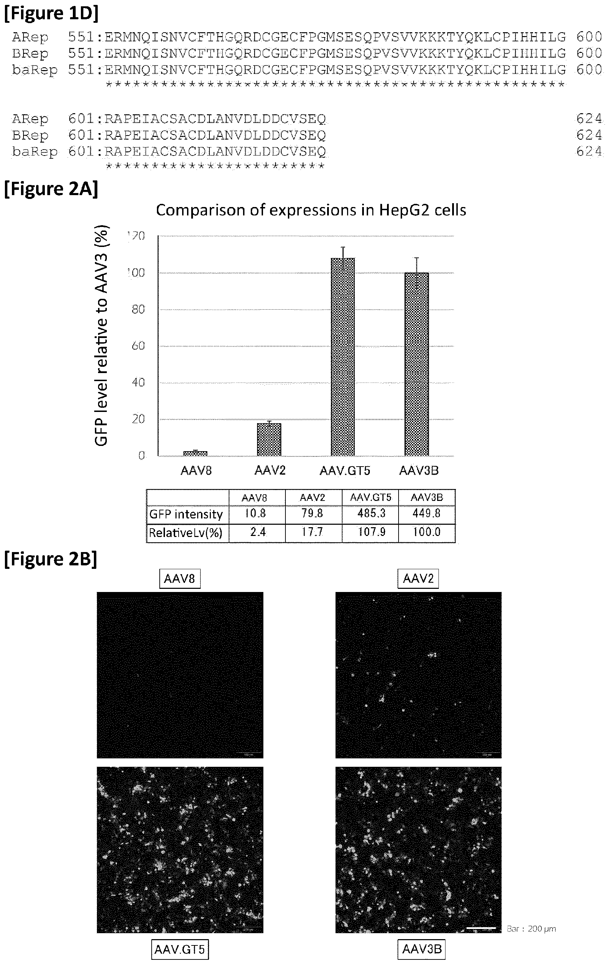

The alignments of the amino acid sequences of the VP1 proteins (SEQ ID NOs:2-4) and the amino acid sequences of the Rep proteins (SEQ ID NOs:6-8) of AAV3A, AAV3B, and AAV.GT5 are shown in Figures 1A-1D.

### (2) Confirmation of infectivity of AAV.GT5 to human liver-derived cell line HepG2 and PXB

The infectivity of AAV.GT5 prepared above to human liver-derived HepG2 cells was confirmed.

HepG2 cells were seeded at 5 × 10⁴ cells/well in a 96-well optical bottom plate. The next day, AAV8-CMV-AcGFP, AAV2-CMV-AcGFP, AAV3-CMV-AcGFP and AAVGT5-CMV-AcGFP were each administered at 5 × 10⁸ vg/well. After culturing at 37°C in a 5% CO₂ incubator for seven days, fluorescence intensities of GFP were measured and quantitatively compared in a plate reader (Table 1 and Figure 2A). The appearances of the cells upon the measurement are shown in Figure 2B.

**Table 1: Infectivity of each AAV vector to HepG2 cells**

| | AAV8 | AAV2 | AAV.GT5 | AAV3B |
|---|---|---|---|---|
| GFP intensity | 10.8 | 79.8 | 485.3 | 449.8 |
| Relative value (%) | 2.4 | 17.7 | 107.9 | 100.0 |

According to the results of the GFP intensity measurements, AAV.GT5 showed an approximately 1.1 times (107.9%) higher expression level than AAV3B. Therefore, AAV.GT5 is considered to be capable of transferring a gene into the human liver-derived cells at a level comparable to or higher than the gene transfer level with AAV3. On the other hand, AAV8 and AAV2 show lower efficiencies of gene transfer into human liver-derived cells than the efficiencies of AAV3 and AAV.GT5 (Figure 2A).

In the above measurement system employing GFP expression, both AAV.GT5 and AAV3B were close to the upper limit of the measurement range, and thus the results may have indicated a smaller difference than the actual amount ratio. Therefore, the infectivity of each rAAV vector to human liver-derived cell line HepG2 was confirmed using luciferase. In addition, AAV3B and AAV-LK03 were used as controls.

To HepG2 cells obtained 1 day after seeding the cells at 2 × 10⁴ cells/well, AAV.GT5-CMV-Luciferase, AAV-LK03-CMV-Luciferase, and AAV3B-CMV-Luciferase were each administered, at MOI=800 vg/cell, and the resultant cells were cultured.

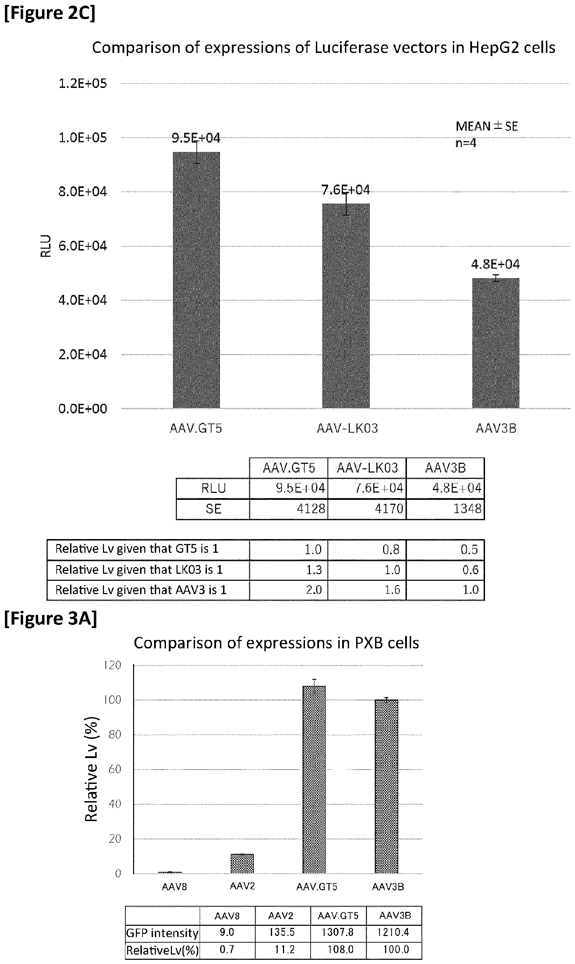

Two days after the AAV administration, luciferase activity was measured using Bright-Glo Luciferase Assay System (Promega) (n=4). The graph and relative values of luminescence intensity (RLU) measurements are shown in Figure 2C.

The RLU measurements confirmed that AAV.GT5 resulted in expression at twice the level of AAV3B and 1.3 times the level of AAV-LK03 (Table 2 and Figure 2C).

**Table 2: Infectivity of each AAV vector to HepG2 cells**

| | | AAV.GT5 | AAV-LK03 | AAV3B |
|---|---|---|---|---|
| | RLU | 9.5E + 04 | 7.6E + 04 | 4.8E + 04 |
| | SE | 4,128 | 4,170 | 1,348 |
| | | | | |
| Relative level given that GT5 is 1 | | 1.0 | 0.8 | 0.5 |
| Relative level given that LK03 is 1 | | 1.3 | 1.0 | 0.6 |
| Relative level given that AAV3 is 1 | | 2.0 | 1.6 | 1.0 |

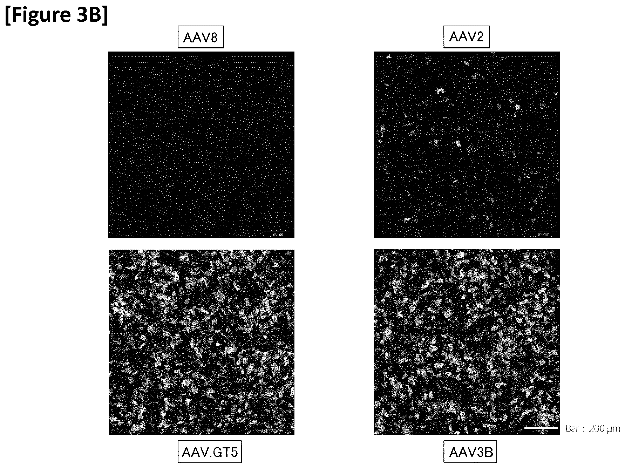

The results of the gene transferring into the PXB cells, which were collected from a PXB mouse liver and composed of 90% or more human hepatocytes, are shown in Figures 3A and 3B. Six days after seeding the PXB cells (PhoenixBio Co., Ltd.) at 7 × 10⁴ cells/well in a 96-well plate, AAV8-CMV-AcGFP, AAV2-CMV-AcGFP, AAV3-CMV-AcGFP, and AAVGT5-CMV-AcGFP were each administered to the cells at 5 × 10⁸ vg/well. After culturing the resultant cells at 37°C in a 5% CO₂ incubator for 7 days, fluorescence intensities of GFP were measured and quantitatively compared in a plate reader (Table 3 and Figure 3A). The appearances of the cells upon this measurement are shown in Figure 3B.

**Table 3: Infectivity of each AAV vector to PXB cells**

| | AAV8 | AAV2 | AAV.GT5 | AAV3B |
|---|---|---|---|---|
| GFP intensity | 9.0 | 135.5 | 1307.8 | 1210.4 |
| Relative value (%) | 0.7 | 11.2 | 108.0 | 100.0 |

As was the case with HepG2 cells, the GFP expression level of AAV.GT5-CMV-AcGFP was again about 1.1 times higher than the level of AAV3B in PXB cells.

Hence, AAV.GT5 is considered to be capable of transferring a gene into these human liver-derived cells at a level comparable to or higher than that by gene transfer with AAV3B. On the other hand, AAV8 and AAV2 shows lower efficiencies of gene transfer into human hepatocytes than the efficiencies of AAV3 and AAV.GT5 (Figures 2 and 3).

### (3) Measurement of antibody titers of neutralizing antibodies

Following the procedure described in (e) above, 10 types of sera (Sera #01-#10) were used to obtain maximum dilutions (neutralizing antibody titers) that can reduce the luciferase activity using AAV.GT5, AAV-LK03, AAV3B, and AAV2 to 50% or less. These results are shown in Table 4.

Regarding neutralizing antibody titers, AAV.GT5 showed the lowest titer among the tested 10 sera, i.e, AAV.GT5 showed the weakest reactivity with the antibodies. The neutralizing antibody titers of AAV.GT5 were about 1/8-1/4 of the titers of AAV-LK03 and AAV3B.

**Table 4. Neutralizing antibody titer**

| | AAV.GT5 | AAV-LK03 | AAV3B | AAV2 |
|---|---|---|---|---|
| Serum #01 | 1:8 | **1:32** | **1:32** | **1:64** |
| Serum #02 | 1:32 | **1:128≤** | **1:128≤** | **1:64** |
| Serum #03 | 1:4 | 1:16 | **1:32** | **1:32** |
| Serum #04 | 1:16 | **1:64** | **1:64** | **1:64** |
| Serum #05 | 1:16 | **1:64** | **1:64** | **1:64** |
| Serum #06 | 1:16 | **1:64** | **1:64** | **1:64** |
| Serum #07 | 1:16 | **1:64** | **1:64** | **1:64** |
| Serum #08 | 1:16 | **1:64** | **1:64** | **1:128≤** |
| Serum #09 | 1:8 | **1:32** | **1:32** | **1:64** |
| Serum #10 | **1:128** | **1:256≤** | **1:256≤** | **1:128** |

| | | | | |
|---|---|---|---|---|
| *Antibody titers of 1:32 or greater were considered as positive and shown in bold | | | | |

Based on the results of the above neutralizing antibody titer measurement, for example, in the case of Serum #01, the controls showed the neutralizing antibody titers of 1:32 or 1:64 whereas AAV.GT5 showed 1:8, indicating about 4 times or more improvement in the neutralizing antibody titer.

### (4) Effect of antiserum on inhibition of expression

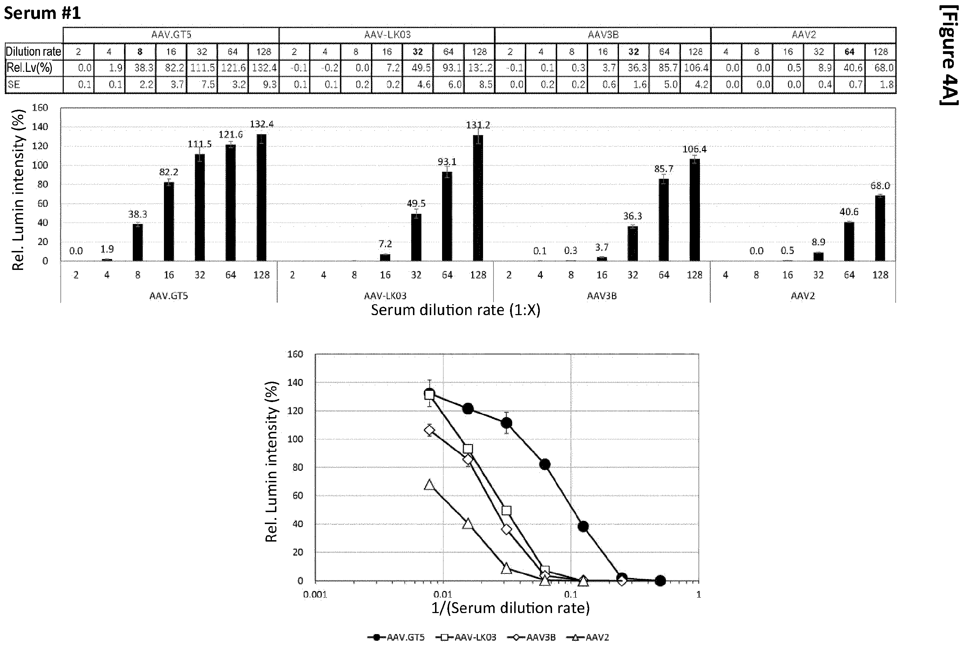

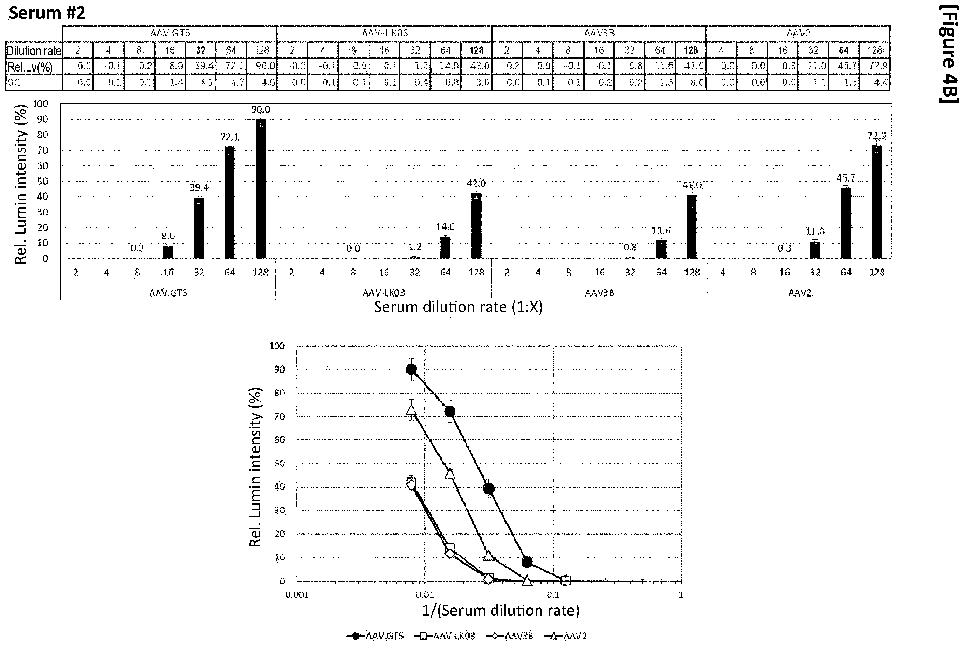

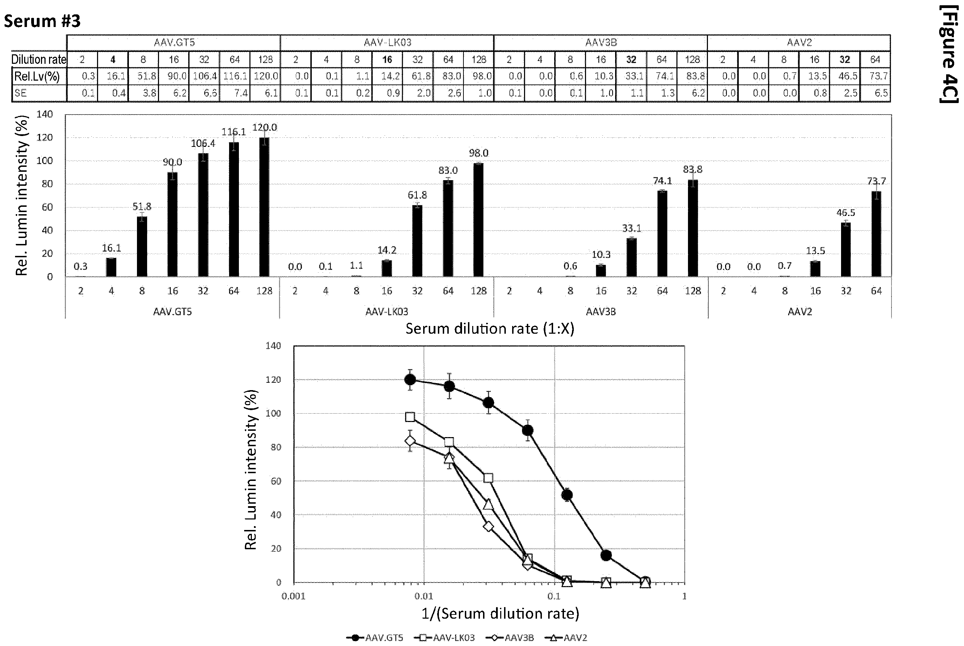

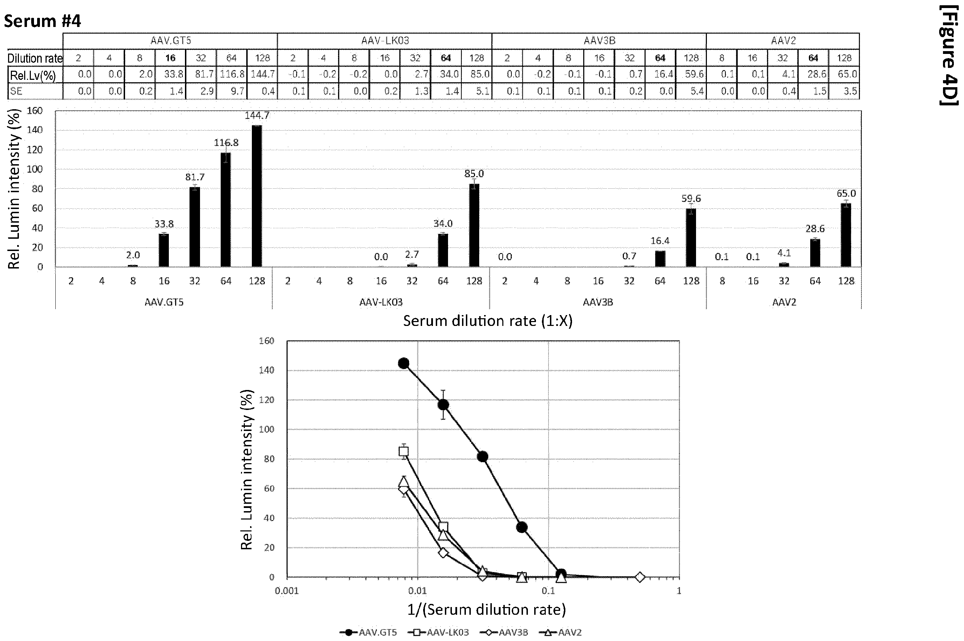

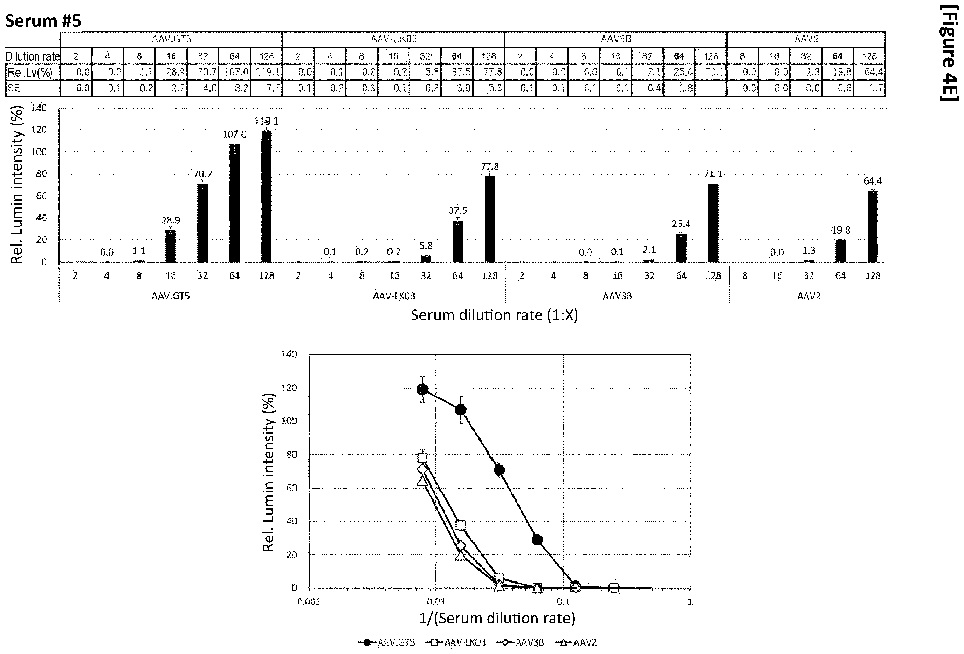

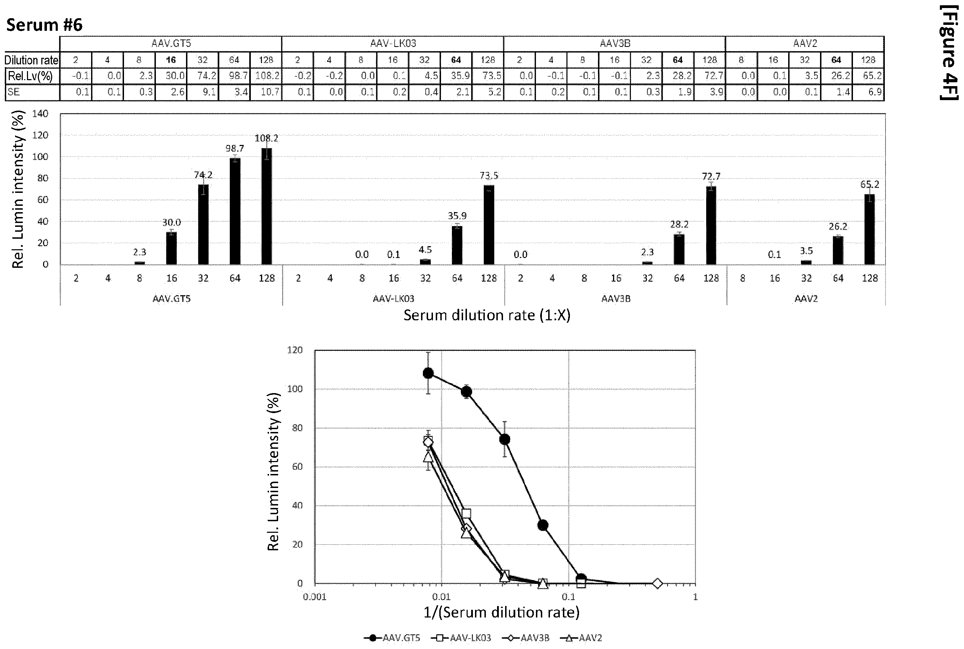

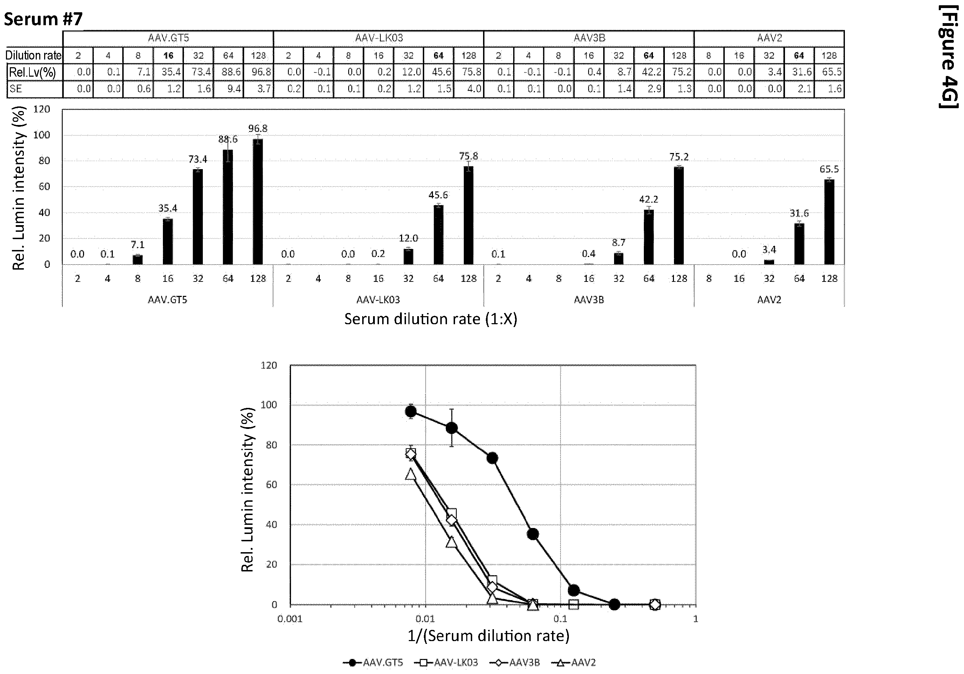

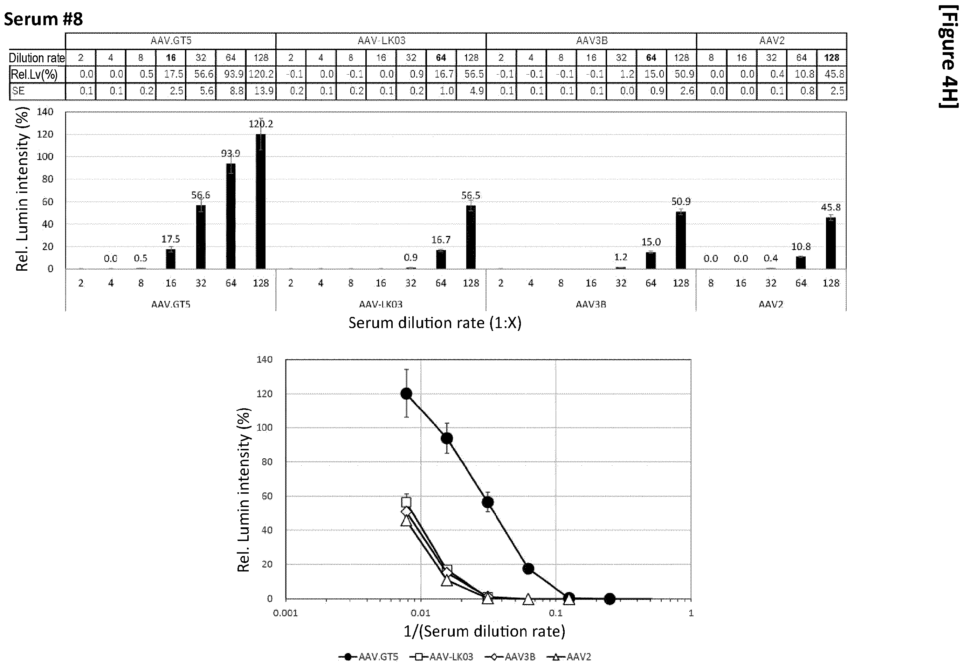

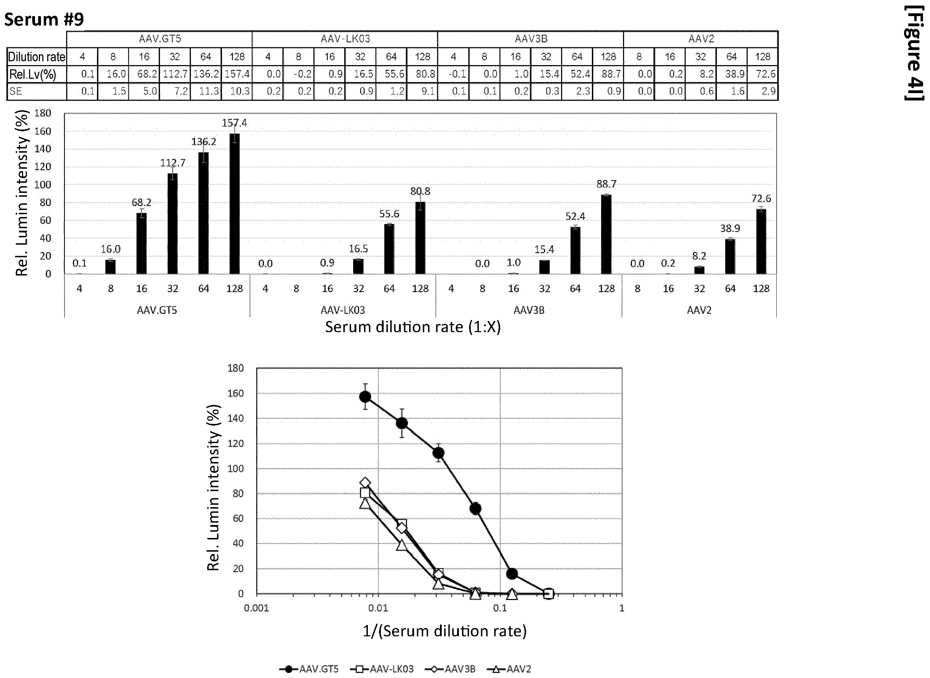

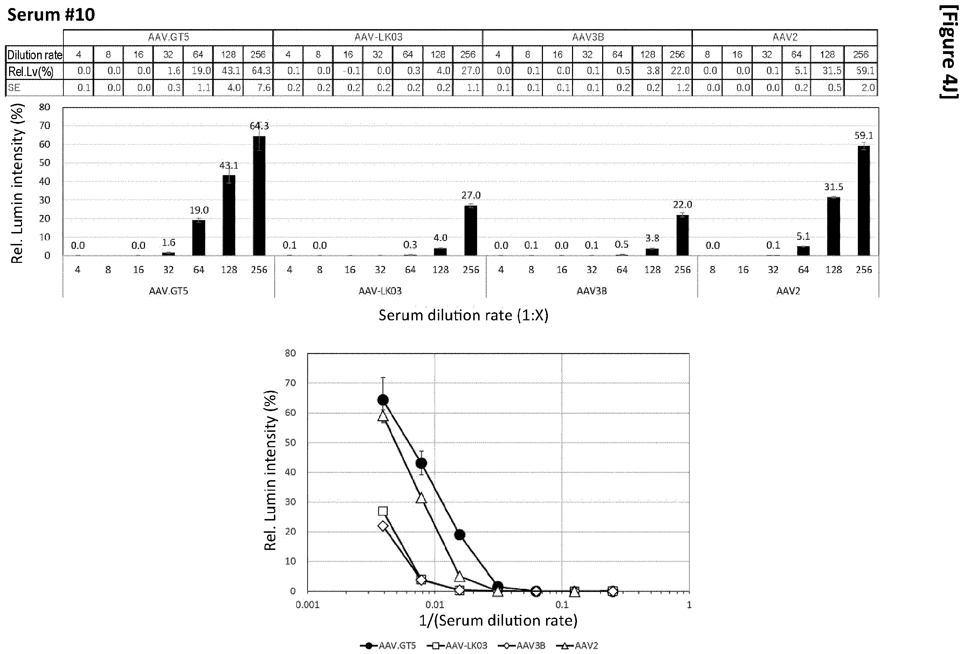

In addition, based on the results of the above neutralizing antibody titer measurements, the gene expression levels using AAV.GT5, AAV-LK03, AAV3B, and AAV2 under the influence of neutralizing antibodies were compared. The results are shown in Figures 4A-4J.

The approximately 4 times or more improvement in the neutralizing antibody titers described above also resulted in a very large difference in the expression levels when compared at the same serum dilution rate.

For example, when luciferase activities were compared in the presence of sera at the same serum dilution (e.g., 32-fold dilution), AAV.GT5 produced very large difference, e.g., 4-fold or more, compared to wild-type AAV3B and AAV2 (see tables and middle graphs in Figures 4A-4J).

In addition, AAV.GT5 also showed that the amount of antiserum (for example, in the case of using Serum #02) required for the same luciferase activity level (e.g., half of the maximum activity level) was 1.5 times higher than that of AAV-LK03, wild-type AAV3B, and AAV2 (see lower graphs in Figures 4A-4J).

These results indicate that AAV.GT5 was least susceptible to inhibition of gene expression by neutralizing antibodies than any of the controls, i.e., AAV-LK03, AAV3B, and AAV2.

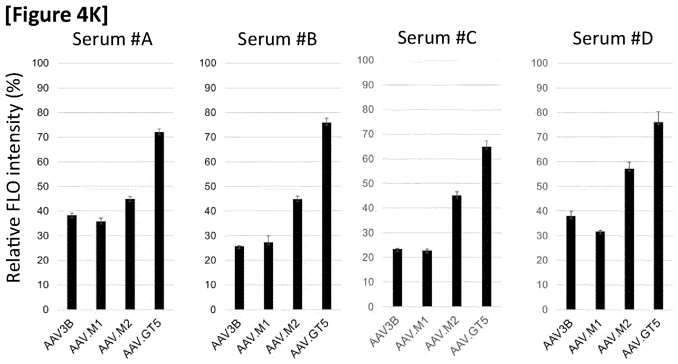

Following the procedure described in (e) above except using GFP as a reporter, 32-fold dilutions of four sera (Sera #A, #B, #C and #D) were used to confirm suppressive inhibition of GFP expression by AAV.GT5, AAV.M1 (S587A), AAV.M2 (S472A + S587A), AAV3B, and AAV2. These results are shown in Figure 4K.

These results indicate that AAV.GT5 was least susceptible to inhibition of expression by neutralizing antibodies than any of the controls, i.e., AAV.M1, AAV.M2, AAV3B, and AAV2.

### (5) Preparation of OTC expression vector

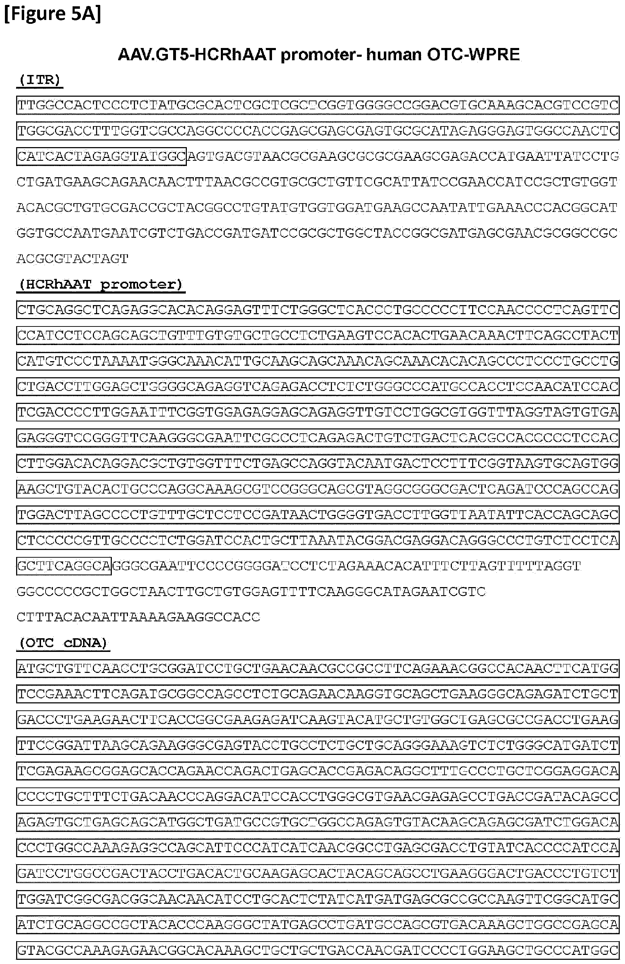

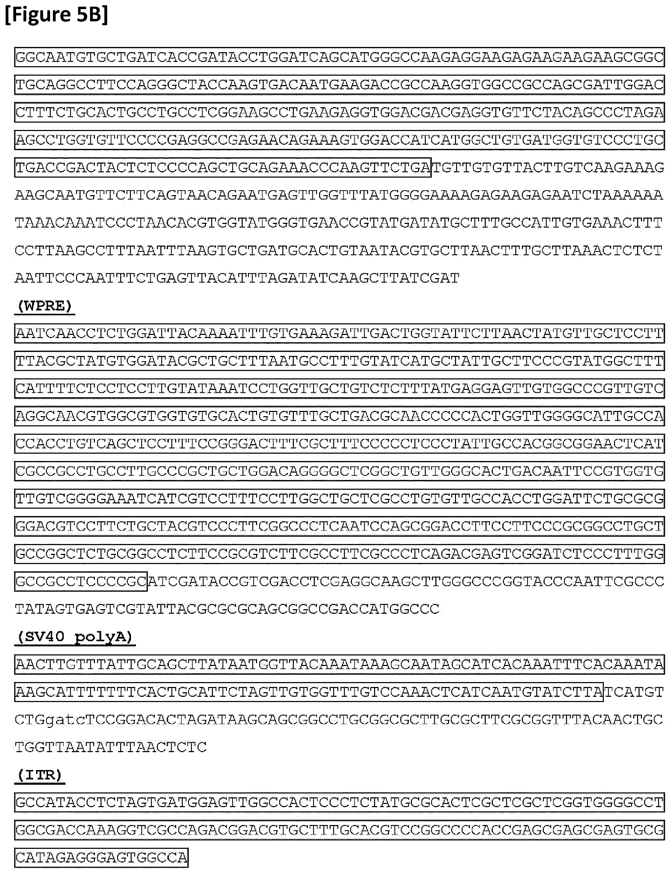

For the expression of recombinant OTC using AAV.GT5, DNA having the following configuration (referred to as AAV.GT5-OTC) was prepared. More detailed sequence configuration is shown in Figure 5A, Figure 5B, and SEQ ID NO: 12. 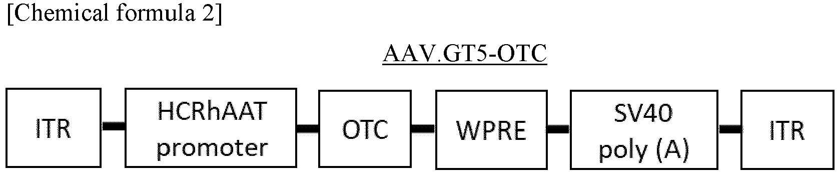
ITR: AAV inverted terminal repeat
HCRhAAT: Chimeric promoter, enhancer element in the liver regulatory region of the Apo E/C1 gene, and human antitrypsin promoter
WPRE: WPRE sequence from Woodchuck hepatitis B virus
OTC: cDNA of human ornithine transcarbamylase

### (6) Comparison of OTC expressions of r AAV vectors

AAV.GT5-OTC was compared with AAV8-OTC in terms of infection to human hepatocytes and expression efficiency using PXB cells which were collected from PXB mouse liver and composed of 90% or more human hepatocytes.

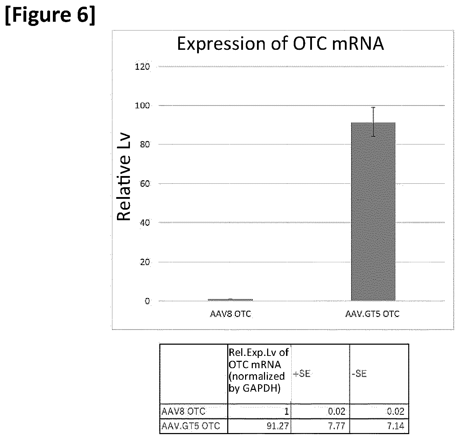

The comparative results of the OTC mRNA expression level of AAV.GT5 are shown in Table 5 and Figure 6.

**Table 5**

| | Relative OTC mRNA expression level (normalized by GAPDH) | +SE | -SE |
|---|---|---|---|
| AAV8 OTC | 1 | 0.02 | 0.02 |
| AAV.GT5 OTC | 91.27 | 7.77 | 7.14 |

It is confirmed that AAV.GT5 showed an approximately 90-fold or more increase in the OTC mRNA expression level as compared with the control.

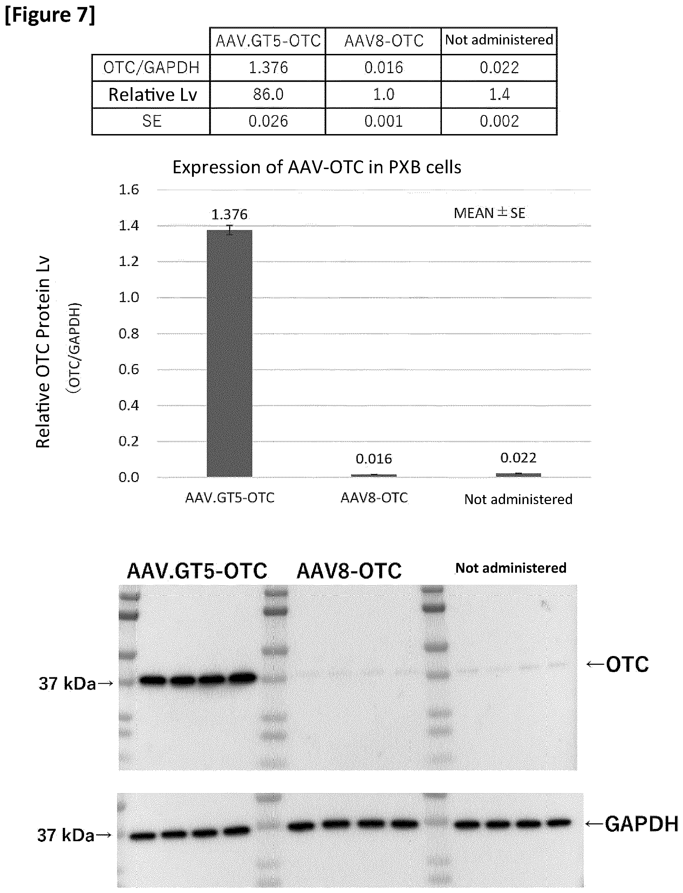

In addition, the comparative results of OTC protein expression level of AAV.GT5 are shown in Table 6 and Figure 7.

**Table 6**

| | AAV.GT5-OTC | AAV8-OTC | Not administered |
|---|---|---|---|
| OTC/GAPDH | 1.376 | 0.016 | 0.022 |
| Relative value | 86.0 | 1.0 | 1.4 |
| SE | 0.026 | 0.001 | 0.002 |

Similar to the results of mRNA expression levels, it is confirmed that AAV.GT5 was showed an approximately 86-fold or more increase in the protein expression level as compared with the control.

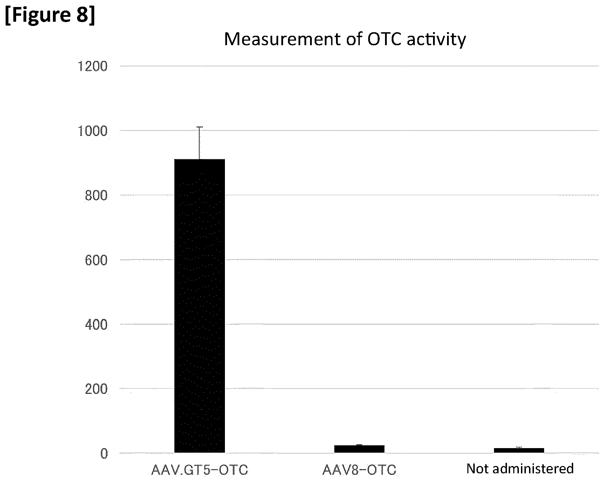

Furthermore, the comparative results of OTC enzymatic activity of AAV.GT5 are shown in Table 7 and Figure 8.

**Table 7**

| | AAV.GT5-OTC | AAV8-OTC | Not administered |
|---|---|---|---|
| OTC activity | 910.9 | 24.3 | 15.4 |
| SE | 100.1 | 1.6 | 2.9 |

As can be appreciated from the above results, it is expected that AAV.GT5-OTC can be used as a therapeutic means to provide advantages such as very strong OTC expression and/or a large reduction in virus vector dose.

### (7) Verification of reduction in ammonia concentration by AAV.GT5-OTC

Hepatocytes, etc. obtained from an OTC-deficient patient are cultured, and AAV.GT5-OTC at various concentrations and a control vector and NH₄Cl are each added thereto to monitor the ammonia concentrations in the culture media over time.

Alternatively, hepatocytes obtained from an OTC-deficient patient are used to generate an animal model with OTC deficiency (see, for example, Sugahara, G. et al., J Inherit Metab Dis. 2020: 1-11). AAV.GT5-OTC and control vectors at various concentrations are administered to the generated animal models to monitor the ammonia levels in blood over time.

### INDUSTRIAL APPLICABILITY

The rAAV vector according to the present invention was able to reduce the attack of neutralizing antibodies of a living body and was able to attain gene transfer into target cells in the presence of the neutralizing antibodies at, for example, approximately 4 times or higher efficiency. Furthermore, under the control of a liver-specific promoter, human ornithine transcarbamylase protein was able to be expressed at approximately 90 times or higher efficiency than the control.

Accordingly, it is considered that the rAAV vector for expressing human ornithine transcarbamylase protein of the present invention can be used to attain more efficient treatment of ornithine transcarbamylase deficiency.

### SEQUENCE LISTING FREE TEXT

SEQ ID NO:1: Amino acid sequence of AAV2 capsid protein
SEQ ID NO:2: Amino acid sequence of AAV3A capsid protein
SEQ ID NO:3: Amino acid sequence of AAV3B capsid protein
SEQ ID NO:4: Amino acid sequence of AAV.GT5 modified capsid protein (S472A, S587A, N706A)
SEQ ID NO:5: Amino acid sequence of AAV8 capsid protein
SEQ ID NO:6: Amino acid sequence of AAV3A Rep protein (ARep)
SEQ ID NO:7: Amino acid sequence of AAV3B Rep protein (BRep)
SEQ ID NO:8: Amino acid sequence of AAV.GT5 Rep protein (baRep)
SEQ ID NO:9: Amino acid sequence of AAV-LK03 capsid protein
SEQ ID NO:10: cDNA sequence of ornithine transcarbamylase (OTC)
SEQ ID NO:11: Amino acid sequence of ornithine transcarbamylase (OTC)
SEQ ID NO:12: Sequence of OTC expression vector (AAV.GT5-HCRhAAT promoter-human OTC-WPRE)
SEQ ID NO:13: OTC Forward primer for detection of OTC mRNA expression
SEQ ID NO:14: OTC Reverse primer for detection of OTC mRNA expression
SEQ ID NO: 15: GAPDH Forward primer for detection of GAPDH mRNA expression
SEQ ID NO: 16: GAPDH Reverse primer for detection of GAPDH mRNA expression
SEQ ID NO: 17: Amino acid sequence of modified AAV.M1 capsid protein (S587A)
SEQ ID NO: 18: Amino acid sequence of modified AAV.M2 capsid protein (S472A, S587A)

## Claims

1. An adeno-associated virus vector comprising:
a capsid protein containing an amino acid sequence in which at least one of serine at position 472, serine at position 587, and asparagine at position 706 in the amino acid sequence represented by SEQ ID NO:2 or 3 is substituted with another amino acid, or an amino acid sequence in which 1-6 residues in the substituted amino acid sequence are deleted, substituted, inserted, and/or added, in addition to the residues at positions 472, 587, and 706; and
a polynucleotide encoding a protein that contains the amino acid sequence represented by SEQ ID NO:11 or an amino acid sequence having 90% or more identity to the amino acid sequence represented by SEQ ID NO:11, and that has ornithine transcarbamylase activity,
wherein the adeno-associated virus vector does not cross-react at least with a neutralizing antibody against AAV serotype 2.

2. The adeno-associated virus vector according to claim 1, comprising a capsid protein having an amino acid sequence in which each of the serine at position 472, the serine at position 587, and the asparagine at position 706 is substituted with an amino acid selected from the group consisting of glycine, alanine, valine, leucine, threonine, and isoleucine.

3. The adeno-associated virus vector according to claim 1, comprising a capsid protein having an amino acid sequence in which at least one of the serine at position 472, the serine at position 587, and the asparagine at position 706 is substituted with alanine.

4. The adeno-associated virus vector according to claim 1, wherein the capsid protein comprises a protein having the amino acid sequence represented by SEQ ID NO:4.

5. The adeno-associated virus vector according to claim 1, wherein the neutralizing antibody is an antibody against an adeno-associated virus of a serotype different from AAV3 or AAV8.

6. The adeno-associated virus vector according to claim 1, wherein the neutralizing antibody is an antibody against AAV2.

7. The adeno-associated virus vector according to claim 1, comprising a virus genome containing a hepatocyte-specific promoter sequence.

8. The adeno-associated virus vector according to claim 1, wherein the hepatocyte-specific promoter sequence comprises a promoter selected from the group consisting of an ApoE promoter, an antitrypsin promoter, a cKit promoter, a promoter for a liver-specific transcription factor (HNF-1, HNF-2, HNF-3, HNF-6, C/ERP or DBP), an albumin promoter, a promoter for a thyroxine-binding globulin (TBG), and a HCRhAAT promoter, or a promoter which contains a polynucleotide sequence having 90% or more homology with the promoters and which functions in a liver-specific manner.

9. An adeno-associated virus vector comprising:
a capsid protein containing an amino acid sequence in which at least one of serine at position 472, serine at position 587, and asparagine at position 706 in the amino acid sequence represented by SEQ ID NO:2 or 3 is substituted with another amino acid, or an amino acid sequence in which 1-6 residues in the substituted amino acid sequence are deleted, substituted, inserted, and/or added, in addition to the residues at positions 472, 587, and 706; and
a polynucleotide encoding a protein that contains the amino acid sequence represented by SEQ ID NO:11 or an amino acid sequence having 90% or more identity to the amino acid sequence represented by SEQ ID NO:11, and that has ornithine transcarbamylase activity.

10. A polynucleotide, encoding any one of the following sequences:
an amino acid sequence in which at least one of serine at position 472, serine at position 587, and asparagine at position 706 in the amino acid sequence represented by SEQ ID NO:2 or 3 is substituted with another amino acid, and 1-6 amino acid residues at the other residue positions are deleted, substituted, inserted, or added;
an amino acid sequence in which each of the serine at position 472, the serine at position 587, and the asparagine at position 706 is substituted with an amino acid selected from the group consisting of glycine, alanine, valine, leucine, threonine, and isoleucine;
an amino acid sequence in which at least one of the serine at position 472, the serine at position 587, and the asparagine at position 706 is substituted with alanine; and
the amino acid sequence represented by SEQ ID NO:4.

11. A pharmaceutical composition for transferring a gene into the liver of a living body, comprising the adeno-associated virus vector according to any one of claims 1 to 9.

12. The pharmaceutical composition according to claim 11, wherein the living body is human.

13. The pharmaceutical composition according to claim 11 or 12, which is used for the treatment of ornithine transcarbamylase deficiency.
